# EUROPEAN PATENT SPECIFICATION

(11) **EP 2 968 219 B1**
(45) Date of publication and mention of the grant of the patent: **24.04.2019**
(21) Application number: 14712924.1
(22) Date of filing: 05.03.2014
(51) Int. Cl.: A61K 31/122, A61K 31/4188, A61K 45/08, A61P 35/00

(54) **NOVEL THERAPY FOR PROSTATE CARCINOMA**
NEUARTIGE THERAPIE GEGEN PROSTATAKARZINOME
NOUVELLE THÉRAPIE POUR LE CARCINOME DE LA PROSTATE

(30) Priority: 14.03.2013 US 201361785982 P
(43) Date of publication of application: 20.01.2016
(73) Proprietor: Pellficure Pharmaceuticals, Inc., La Jolla, CA 92037 (US)
(72) Inventor: BORGSTROM, Per, La Jolla, CA 92037 (US)
(74) Representative: Plougmann Vingtoft a/s
(86) International application number: PCT/US2014/020637
(87) International publication number: WO 2014/158875

(56) References cited:
- WO-A1-95/07927
- WO-A1-95/07927
- WO-A1-98/33506
- WO-A1-2012/158884
- WO-A2-2010/091299
- WO-A2-2011/082245
- WO-A2-2012/018948
- WO-A2-2012/058529
- WO-A2-2012/064943
- TINDALL D J ET AL: "The Rationale for Inhibiting 5alpha-Reductase Isoenzymes in the Prevention and Treatment of Prostate Cancer", JOURNAL OF UROLOGY, LIPPINCOTT WILLIAMS & WILKINS, BALTIMORE, MD, US, vol. 179, no. 4, 1 April 2008 (2008-04-01) , pages 1235-1242, XP022618475, ISSN: 0022-5347, DOI: 10.1016/J.JURO.2007.11.033 [retrieved on 2008-02-20]
- PARISA ABEDINPOUR ET AL: "The combination of plumbagin with androgen withdrawal causes profound regression of prostate tumors in vivo", THE PROSTATE, [Online] vol. 73, no. 5, 19 September 2012 (2012-09-19), pages 489-499, XP055115395, ISSN: 0270-4137, DOI: 10.1002/pros.22585
- Jacoby et al: "Differential effects of galeterone abiraterone, abiraterone, orteronel and ketoconazole on CYP 17 and steroidogenesis.", Urotoday.com , 25 February 2013 (2013-02-25), XP002723945, Retrieved from the Internet: URL:http://www.urotoday.com/GU-Cancers-Sym p.-2013-Prostate-Cancer/gu-cancers-symposi um-2013-differential-effects-of-galeterone -abiraterone-orteronel-and-ketoconazole-on -cyp17-and-steroidogenesis-by-douglas-b-ja coby-et-al-session.html [retrieved on 2014-05-02]
- YE LEPING ET AL: "Inhibitors of testosterone biosynthetic and metabolic activation enzymes.", MOLECULES (BASEL, SWITZERLAND) 2011, vol. 16, no. 12, 2011, pages 9983-10001, XP002723946, ISSN: 1420-3049
- ERI HASEGAWA ET AL: "Effect of Polyphenols on Production of Steroid Hormones from Human Adrenocortical NCI-H295R Cells", BIOLOGICAL & PHARMACEUTICAL BULLETIN, vol. 36, no. 2, 1 January 2013 (2013-01-01), pages 228-237, XP055115995, ISSN: 0918-6158, DOI: 10.1248/bpb.b12-00627
- TRENT D LUND ET AL: "Equol is a Novel Anti-Androgen that Inhibits Prostate Growth and Hormone Feedback", BIOLOGY OF REPRODUCTION, NEW YORK, NY [U.A.] : ACADEM. PRESS, US, vol. 70, no. 4, 1 April 2004 (2004-04-01), pages 1188-1195, XP003024362, ISSN: 0006-3363, DOI: 10.1095/BIOLREPROD.103.023713 [retrieved on 2003-12-17]
- TINDALL D J ET AL: "The Rationale for Inhibiting 5alpha-Reductase Isoenzymes in the Prevention and Treatment of Prostate Cancer", JOURNAL OF UROLOGY, LIPPINCOTT WILLIAMS & WILKINS, BALTIMORE, MD, US, vol. 179, no. 4, 1 April 2008 (2008-04-01) , pages 1235-1242, XP022618475, ISSN: 0022-5347, DOI: 10.1016/J.JURO.2007.11.033 [retrieved on 2008-02-20]

## Description

### FIELD OF THE INVENTION

Aspects of the present application relate to the fields of chemistry, biochemistry and medicine. More particularly, disclosed herein are combination therapies, wherein a naphthoquinone analog, such as plumbagin, and a hormone therapy agent are provided to a subject having a cancer, such as a prostate cancer.

### BACKGROUND OF THE INVENTION

Prostate cancer develops in the prostate and is typically slow growing; however, some prostate cancers are aggressive. Prostate cancer cells are typically androgen/testosterone/DHT dependent and may metastasize from the prostate to other parts of the body, particularly the bones and lymph nodes. Treatment options for prostate cancer that remains within the prostate include watchful waiting/active surveillance, external beam radiation therapy, brachytherapy, cryosurgery, high-intensity focused ultrasound (HIFU), and surgery. Hormonal therapy and chemotherapy are often reserved for disease that has spread beyond the prostate. However, there are exceptions in that radiation therapy may be used for some advanced tumors, and hormonal therapy may be used for some early stage tumors.

After one to three years of hormonal therapy, it is common that prostate cancer cells resume growth despite the androgen/testosterone/DHT blockade. Previously referred to as "hormone-refractory prostate cancer" or "androgen-independent prostate cancer," the term castration-resistant prostate cancer (CRPC) is now commonly used. Chemotherapeutic agents and immunotherapy have been shown to prolong survival after CRPC but the survival benefit is limited. Despite the efforts of many, the need for more cancer treatments, in particular prostate cancer treatments, is manifest.

### SUMMARY

Some embodiments are directed to a method of inhibiting or delaying the growth of androgen-dependent prostate cancer, and/or inhibiting or delaying the onset of castration-resistant prostate cancer (CRPC), comprising: selecting a patient in need of a compound that inhibits or delays the growth of androgen-dependent prostate cancer, and/or in need of a compound that inhibits or delays the onset of castration-resistant prostate cancer (CRPC), wherein said patient is also selected as one in need of a compound that reduces or inhibits androgen biosynthesis; and administering to said patient a therapeutically effective amount of a compound of Formula (I), or a pharmaceutically acceptable salt of Formula (I): 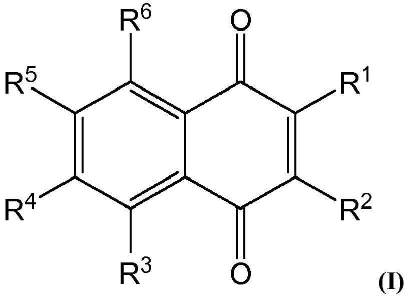 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl; wherein the compound of Formula (I) is administered to the patient before, during, or after administration of a compound that reduces or inhibits androgen biosynthesis in said patient; and wherein the compound that reduces or inhibits androgen biosynthesis is a selective inhibitor of the 17,20-lyase activity of cytochrome P450-17 (CYP17).

Some embodiments are directed to a method of inhibiting or delaying the growth of androgen-dependent prostate cancer, and/or inhibiting or delaying the onset of castration-resistant prostate cancer (CRPC), comprising: selecting a patient in need of a compound that inhibits or delays the growth of androgen-dependent prostate cancer, and/or in need of a compound that inhibits or delays the onset of castration-resistant prostate cancer (CRPC); and administering to said patient a therapeutically effective amount of a compound of Formula (I), or a pharmaceutically acceptable salt of Formula (I): 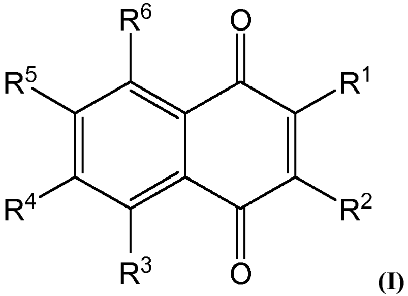 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl; wherein the compound of Formula (I) is administered to the patient before, during, or after administration of a hormonal therapy agent selected from the group consisting of deslorelin, nafarelin, cetrorelix, and ganirelix.

Some embodiments are directed to a method of inhibiting or delaying the growth of androgen-dependent prostate cancer, and/or inhibiting or delaying the onset of castration-resistant prostate cancer (CRPC), comprising: selecting a patient in need of a compound that inhibits or delays the growth of androgen-dependent prostate cancer, and/or in need of a compound that inhibits or delays the onset of castration-resistant prostate cancer (CRPC); and administering to said patient a therapeutically effective amount of a compound of Formula (I), or a pharmaceutically acceptable salt of Formula (I): 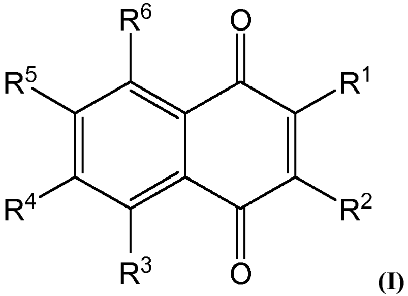 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl; wherein the compound of Formula (I) is administered to the patient before, during, or after administration of a compound selected from the group consisting of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); VT-464; orteronel (also known as "Tak-700"); aminoglutethimide; prochloraz (N-propyl-N-[2-(2,4,6-trichlorophenoxy)ethyl]imidazole-1-carboxamide); dutasteride (5a, 17B)-N-{2,5 bis(trifluoromethyl) phenyl}-3-oxo-4-azaandrost-1-ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18B-glycerrhetinic acid; altraric acid; N-butylbenzene-sulfonamide; and 3,3'-diindolylmethane.

Some embodiments are directed to a method of inducing cell cycle entry, mitosis, or apoptosis of androgen-dependent cancer cells in a patient comprising: reducing androgen biosynthesis in said patient by providing said patient a selective inhibitor of the 17,20-lyase activity of cytochrome P450-17 (CYP17); and (b) providing to said patient an effective amount of a compound of Formula (I), or a pharmaceutically acceptable salt of Formula (I): 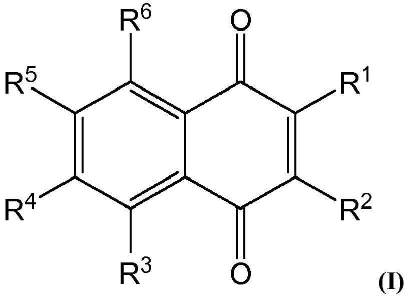
wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²;
R⁵ is selected from the group consisting of hydrogen, an optionally substituted C₁₋₆ alkyl, and -OR¹³; R⁶ is selected from the group consisting of hydrogen, an optionally substituted C₁₋₆ alkyl, and -OR¹⁴; and P,⁷, R⁸, R⁹, R¹⁰, R¹¹, R¹², R¹³, and R¹⁴ are independently selected from hydrogen and an optionally substituted C₁₋₆ alkyl.

Some embodiments are directed to a method of inducing cell cycle entry, mitosis, or apoptosis of androgen-dependent cancer cells in a patient comprising: reducing androgen biosynthesis in said patient by providing said patient a hormonal therapy agent selected from the group consisting of deslorelin, nafarelin, cetrorelix, and ganirelix; and (b) providing to said patient an effective amount of a compound of Formula (I), or a pharmaceutically acceptable salt of Formula (I): 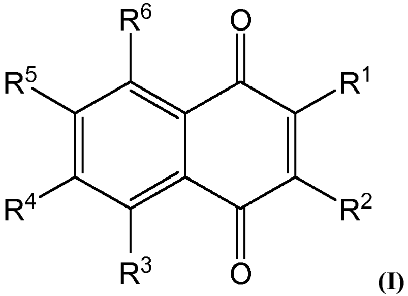 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²;

R⁵ is selected from the group consisting of hydrogen, an optionally substituted C₁₋₆ alkyl, and -OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a method of inducing cell cycle entry, mitosis, or apoptosis of androgen-dependent cancer cells in a patient comprising: providing said patient a compound selected from the group consisting of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); VT-464; orteronel; aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl}-3-oxo-4-azaandrost-1-ene-17-carboxamide); izonteride ((4aR,10bR)-8-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18B-glycerrhetinic acid; altraric acid; N-butylbenzene-sulfonamide; and 3,3'-diindolylmethane; and (b) providing to said patient an effective amount of a compound of Formula (I), or a pharmaceutically acceptable salt of Formula (I): 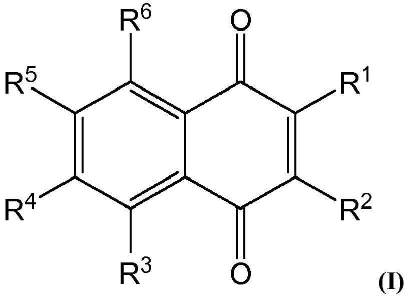 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and -OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a method of making a pharmaceutical that inhibits androgen-dependent prostate cancer cell growth, and/or that inhibits or delays the onset of castration-resistant prostate cancer (CRPC), comprising: (a) providing a pseudo-orthotopic chamber mouse model comprising a mouse with prostate cancer cells in a pseudo-orthotopic chamber; (b) reducing androgen biosynthesis in said mouse by administering a selective inhibitor of the 17,20-lyase activity of cytochrome P450-17 (CYP17); (c) providing the mouse with a compound of Formula (I) or a pharmaceutically acceptable salt thereof: 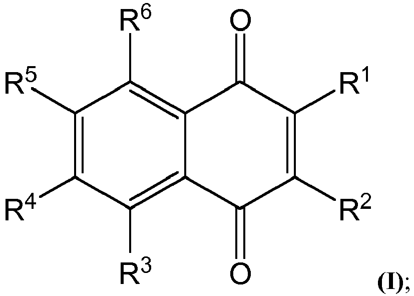 (d) determining whether the compound is effective in inhibiting the growth of androgen-dependent prostate cancer cells, or determining whether the compound is effective in inhibiting or delaying the onset of castration resistant prostate cancer (CRPC); and (e) formulating said compound into a pharmaceutical for use in inhibiting the growth of androgen-dependent prostate cancer cells or inhibiting or delaying the onset of castration resistant prostate cancer (CRPC) wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and -OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a method of making a pharmaceutical that inhibits androgen-dependent prostate cancer cell growth, and/or that inhibits or delays the onset of castration-resistant prostate cancer (CRPC), comprising: (a) providing a pseudo-orthotopic chamber mouse model comprising a mouse with prostate cancer cells in a pseudo-orthotopic chamber; (b) reducing androgen biosynthesis in said mouse by administering a hormonal therapy agent selected from the group consisting of deslorelin, nafarelin, cetrorelix, and ganirelix; (c) providing the mouse with a compound of Formula (I) or a pharmaceutically acceptable salt thereof: 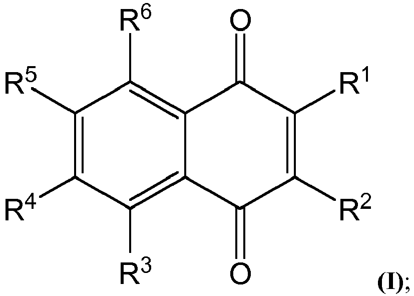 (d) determining whether the compound is effective in inhibiting the growth of androgen-dependent prostate cancer cells, or determining whether the compound is effective in inhibiting or delaying the onset of castration resistant prostate cancer (CRPC); and (e) formulating said compound into a pharmaceutical for use in inhibiting the growth of androgen-dependent prostate cancer cells or inhibiting or delaying the onset of castration resistant prostate cancer (CRPC) wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and -OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a method of making a pharmaceutical that inhibits androgen-dependent prostate cancer cell growth, and/or that inhibits or delays the onset of castration-resistant prostate cancer (CRPC), comprising: (a) providing a pseudo-orthotopic chamber mouse model comprising a mouse with prostate cancer cells in a pseudo-orthotopic chamber; (b) providing the mouse a compound selected from the group consisting of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1 -yl]ethan-1-one); L-39 (17-(5'-Isoxazolyl)androsta-4,16-dien-3-one); VT-464; orteronel; aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl} -3-oxo-4-azaandrost-1 -ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18B-glycerrhetinic acid; altraric acid; N-butylbenzene-sulfonamide; and 3,3'-diindolylmethane; and (c) providing the mouse with a compound of Formula (I) or a pharmaceutically acceptable salt thereof: 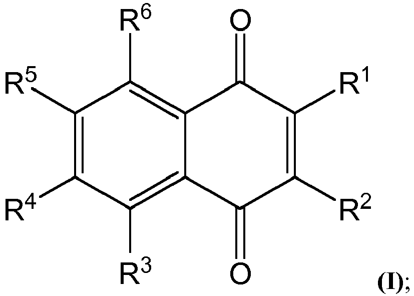 (d) determining whether the compound is effective in inhibiting the growth of androgen-dependent prostate cancer cells, or determining whether the compound is effective in inhibiting or delaying the onset of castration resistant prostate cancer (CRPC); and (e) formulating said compound into a pharmaceutical for use in inhibiting the growth of androgen-dependent prostate cancer cells or inhibiting or delaying the onset of castration resistant prostate cancer (CRPC) wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and -OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a product combination that inhibits androgen-dependent prostate cancer cell growth and/or inhibits or delays the onset of castration-resistant prostate cancer (CRPC) in a subject, wherein the product combination comprises: a selective inhibitor of the 17,20-lyase activity of cytochrome P450-17 (CYP17); and a compound of Formula (I) or a pharmaceutically acceptable salt of Formula (I): 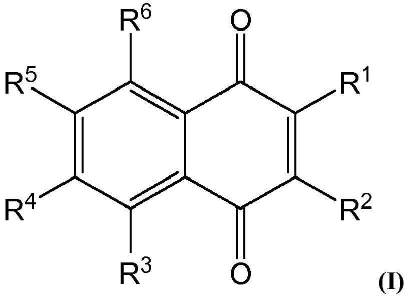 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a product combination that inhibits androgen-dependent prostate cancer cell growth and/or inhibits or delays the onset of castration-resistant prostate cancer (CRPC) in a subject, wherein the product combination comprises: a hormonal therapy agent selected from the group consisting of deslorelin, nafarelin, cetrorelix, and ganirelix; and a compound of Formula (I) or a pharmaceutically acceptable salt of Formula (I): 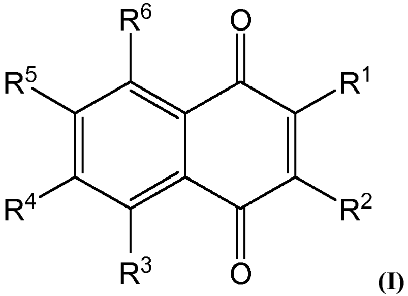 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a product combination that inhibits androgen-dependent prostate cancer cell growth and/or inhibits or delays the onset of castration-resistant prostate cancer (CRPC) in a subject, wherein the product combination comprises: a first compound selected from the group consisting of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); VT-464; orteronel; aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl} -3-oxo-4-azaandrost-1 -ene-17-carboxamide); izonteride ((4aR,10bR)-8-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18B-glycerrhetinic acid; altraric acid; N-butylbenzene-sulfonamide; and 3,3'-diindolylmethane; and a compound of Formula (I) or a pharmaceutically acceptable salt of Formula (I): 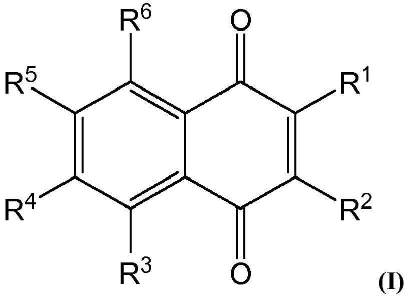 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a compound of Formula (I), or a pharmaceutically acceptable salt of Formula (I), for use in treating or inhibiting the growth of androgen-dependent prostate cancer in a patient, and/or inhibiting or delaying the onset of castration-resistant prostate cancer (CRPC) in a patient, wherein the compound of Formula (I) has the structure: 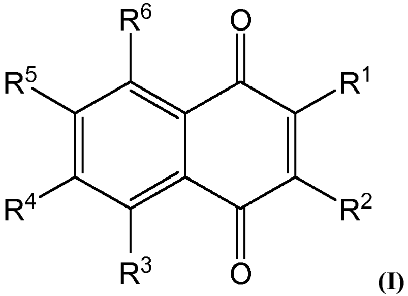 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl; wherein the compound of Formula (I) is administered to the patient before, during, or after administration of a compound that reduces or inhibits androgen biosynthesis in said patient; and wherein the compound that reduces or inhibits androgen biosynthesis is a selective inhibitor of the 17,20-lyase activity of cytochrome P450-17 (CYP17).

Some embodiments are directed to a compound of Formula (I), or a pharmaceutically acceptable salt of Formula (I), for use in treating or inhibiting the growth of androgen-dependent prostate cancer in a patient, and/or inhibiting or delaying the onset of castration-resistant prostate cancer (CRPC) in a patient, wherein the compound of Formula (I) has the structure: 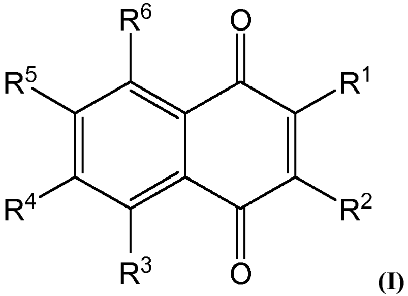 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl; wherein the compound of Formula (I) is administered to the patient before, during, or after administration of a compound that reduces or inhibits androgen biosynthesis in said patient; and wherein the compound that reduces or inhibits androgen biosynthesis is a hormonal therapy agent selected from the group consisting of deslorelin, nafarelin, cetrorelix, and ganirelix.

Some embodiments are directed to a compound of Formula (I), or a pharmaceutically acceptable salt of Formula (I), for use in treating or inhibiting the growth of androgen-dependent prostate cancer in a patient, and/or inhibiting or delaying the onset of castration-resistant prostate cancer (CRPC) in a patient, wherein the compound of Formula (I) has the structure: 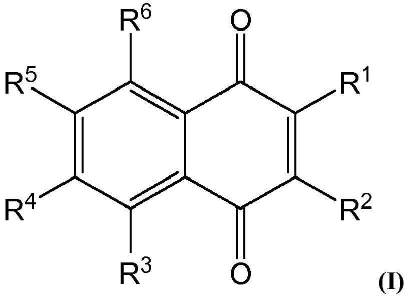 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl; wherein the compound of Formula (I) is administered to the patient before, during, or after administration of a compound selected from the group consisting of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1 -ylmethyl)-1,3 -dioxolan-4-yl]methoxy}phenyl)piperazin-1-yl]ethan-1-one); L-39 (17-(5'-Isoxazolyl)androsta-4,16-dien-3-one); VT-464; orteronel; aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl} -3-oxo-4-azaandrost-1 -ene-17-carboxamide); izonteride ((4aR,10bR)-8-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18B-glycerrhetinic acid; altraric acid; N-butylbenzene-sulfonamide; and 3,3'-diindolylmethane.

Some embodiments are directed to a product combination for use in treating or inhibiting androgen-dependent prostate cancer cell growth and/or inhibiting or delaying the onset of castration-resistant prostate cancer (CRPC) in a subject, wherein the product combination comprises: a selective inhibitor of the 17,20-lyase activity of cytochrome P450-17 (CYP17); and a compound of Formula (I) or a pharmaceutically acceptable salt of Formula (I): 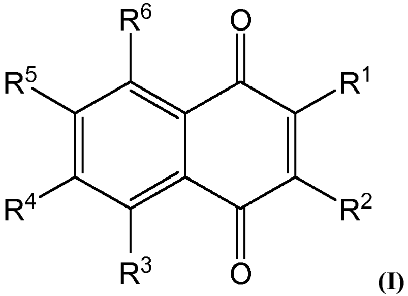 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a product combination for use in treating or inhibiting androgen-dependent prostate cancer cell growth and/or inhibiting or delaying the onset of castration-resistant prostate cancer (CRPC) in a subject, wherein the product combination comprises: a hormonal therapy agent selected from the group consisting of deslorelin, nafarelin, cetrorelix, and ganirelix; and a compound of Formula (I) or a pharmaceutically acceptable salt of Formula (I): 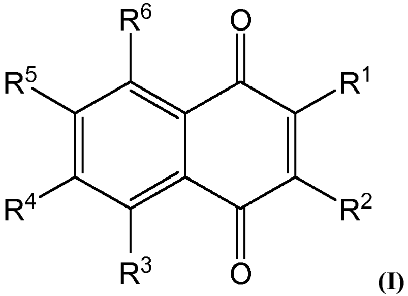 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a product combination for use in treating or inhibiting androgen-dependent prostate cancer cell growth and/or inhibiting or delaying the onset of castration-resistant prostate cancer (CRPC) in a subject, wherein the product combination comprises: a first compound selected from the group consisting of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); VT-464; orteronel; aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl}-3-oxo-4-azaandrost-1-ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[flquinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18β-glycerrhetinic acid; altraric acid; N-butylbenzene-sulfonamide; and 3,3'-diindolylmethane; and a compound of Formula (I) or a pharmaceutically acceptable salt of Formula (I): 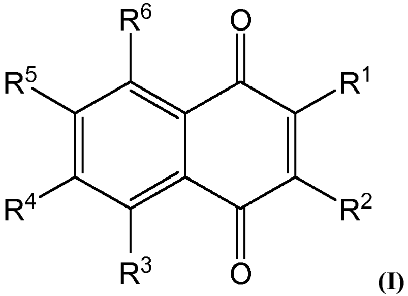 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a use of a compound of Formula (I) and a compound that is a selective inhibitor of the 17,20-lyase activity of cytochrome P450-17 (CYP17) in the manufacture of a medicament for the treatment of androgen-dependent prostate cancer and/or inhibiting or delaying the onset of castration-resistant prostate cancer (CRPC) in a subject wherein the compound of Formula (I) has the structure: 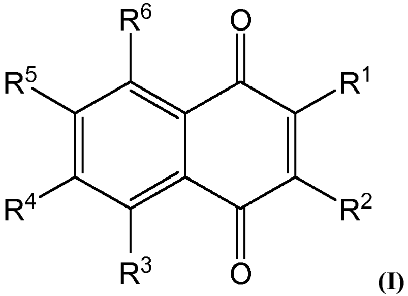 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a use of a compound of Formula (I) and a compound that is a hormonal therapy agent selected from the group consisting of deslorelin, nafarelin, cetrorelix, and ganirelix in the manufacture of a medicament for the treatment of androgen-dependent prostate cancer and/or inhibiting or delaying the onset of castration-resistant prostate cancer (CRPC) in a subject wherein the compound of Formula (I) has the structure: 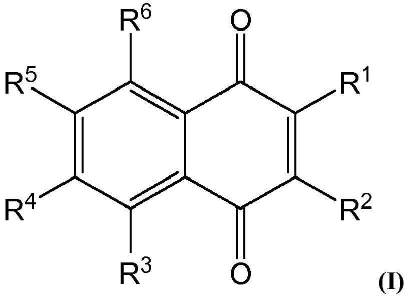 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

Some embodiments are directed to a use of a compound of Formula (I) and a second compound selected from the group consisting of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); VT-464; orteronel; aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl} -3-oxo-4-azaandrost-1 -ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18B-glycerrhetinic acid; altraric acid; N-butylbenzene-sulfonamide; and 3,3'-diindolylmethane in the manufacture of a medicament for the treatment of androgen-dependent prostate cancer and/or inhibiting or delaying the onset of castration-resistant prostate cancer (CRPC) in a subject wherein the compound of Formula (I) has the structure: 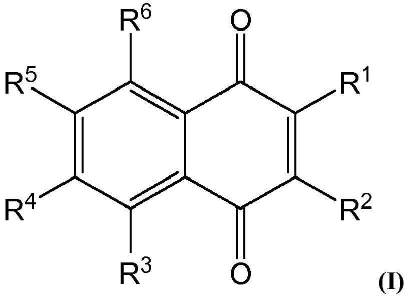 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl.

In some embodiments, the selective inhibitor of the 17,20-lyase activity of CYP17 is selected from the group consisting of orteronel and VT-464. In some embodiments, the selective inhibitor of the 17,20-lyase activity of CYP17 is orteronel. In some embodiments, the selective inhibitor of the 17,20-lyase activity of CYP17 is VT-464.

In some embodiments, the hormonal therapy agent is deslorelin. In some embodiments, the hormonal therapy agent is nafarelin. In some embodiments, the hormonal therapy agent is cetrorelix. In some embodiments, the hormonal therapy agent is ganirelix.

In some embodiments, the compound of Formula (I) is administered to the patient before, during, or after administration of a compound selected from the group consisting of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); and vinclozolin ((RS)-3-(3,5-dichlorophenyl)-5-methyl- 5-vinyloxazolidine-2,4-dione).

In some embodiments, the compound of Formula (I) is administered to the patient before, during, or after administration of a compound selected from the group consisting of galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); VT-464; orteronel; aminoglutethimide; and prochloraz (N-propyl-N-[2-(2,4,6-trichlorophenoxy)ethyl]imidazole-1-carboxamide).

In some embodiments, the compound of Formula (I) is administered to the patient before, during, or after administration of a compound selected from the group consisting of dutasteride (5a, 17β)-N-{2,5 bis(trifluoromethyl) phenyl}-3-oxo-4-azaandrost-1 -ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); and epristeride (17-(tert-butylcarbamoyl)androsta-3,5-diene-3-carboxylic acid).

In some embodiments, the compound of Formula (I) is administered to the patient before, during, or after administration of a compound selected from the group consisting of genisterin; gossypol; equol; 18B-glycerrhetinic acid; altraric acid; N-butylbenzene-sulfonamide; and 3,3'-diindolylmethane.

In some embodiments, R¹, R², R³, R⁴, R⁵ and R⁶ are each hydrogen. In some embodiments, R¹ is methyl; R³ is -OH; and R², R⁴, R⁵ and R⁶ are each hydrogen. In some embodiments, R³ and R⁶ are each -OH; and R¹, R², R⁴ and R⁵ are each hydrogen. In some embodiments, R³ is -OH; and R¹, R², R⁴, R⁵ and R⁶ are each hydrogen. In some embodiments, R¹ and R² are each -SCH₂CH₂OH; and R³, R⁴, R⁵ and R⁶ are each hydrogen. In some embodiments, R¹ and R² are each -OCH³; and R³, R⁴, R⁵ and R⁶ are each hydrogen. In some embodiments, R¹ is -OCH³; and R², R³, R⁴, R⁵ and R⁶ are each hydrogen. In some embodiments, R¹ is methyl; and R², R³, R⁴, R⁵ and R⁶ are each hydrogen. In some embodiments, R¹ and R² are each chloro; and R³, R⁴, R⁵ and R⁶ are each hydrogen. In some embodiments, R¹ is -OH; and R², R³, R⁴, R⁵ and R⁶ are each hydrogen. In some embodiments, R¹ is phytenyl; R² is methyl; and R³, R⁴, R⁵ and R⁶ are each hydrogen. In some embodiments, R¹ and R⁴ are each t-butyl; and R², R³, R⁵ and R⁶ are each hydrogen. In some embodiments, R¹ is -OH; R² is -CH₂-CH=C(CH₃)₂; and R³, R⁴, R⁵ and R⁶ are each hydrogen.

In some embodiments, the compound of Formula (I) is a selected from the group consisting of: the compound where R¹, R², R³, R⁴, R⁵ and R⁶ are each hydrogen; the compound where R³ and R⁶ are each -OH; and R¹, R², R⁴ and R⁵ are each hydrogen; the compound where R³ is -OH; and R¹, R², R⁴, R⁵ and R⁶ are each hydrogen; the compound where R¹ and R² are each -SCH₂CH₂OH; and R³, R⁴, R⁵ and R⁶ are each hydrogen; the compound where R¹ and R² are each -OCH³; and R³, R⁴, R⁵ and R⁶ are each hydrogen; the compound where R¹ is -OCH³; and R², R³, R⁴, R⁵ and R⁶ are each hydrogen; the compound where R¹ is methyl; and R², R³, R⁴, R⁵ and R⁶ are each hydrogen; the compound where R¹ and R² are each -Cl; and R³, R⁴, R⁵ and R⁶ are each hydrogen; the compound where R¹ is -OH; and R², R³, R⁴, R⁵ and R⁶ are each hydrogen; the compound where R¹ is phytenyl; R² is methyl; and R³, R⁴, R⁵ and R⁶ are each hydrogen; the compound where R¹ and R⁴ are each t-butyl; and R², R³, R⁵ and R⁶ are each hydrogen; and the compound where R¹ is -OH; R² is -CH₂-CH=C(CH₃)₂; and R³, R⁴, R⁵ and R⁶ are each hydrogen.

In some embodiments, glucocorticoids are not further administered to the patient.

In some embodiments, the patient's serum testosterone level is reduced to about 5-20%, 10-30%, 20-40%, 30-50%, 40-60%, or 50-70% that of a healthy male subject.

In some embodiments, said method inhibits the growth of prostate cancer.

In some embodiments, said method inhibits or delays the onset of castration-resistant prostate cancer.

In some embodiments, the method further comprises classifying said subject as a member of a population that is at risk for developing CRPC.

In some embodiments, induced cell cycle entry or mitosis results in inducement of apoptosis.

In some embodiments, the method further comprises measuring cell cycle entry, apoptosis, or mitosis of said androgen dependent cancer cells.

In some embodiments, apoptosis is measured by pyknosis.

In some embodiments, the method further comprises identifying said patient for inducing cell cycle entry, mitosis, or apoptosis of androgen dependent cancer cells in said patient.

In some embodiments, said product combination inhibits the growth of prostate cancer in a patient. In some embodiments, said product combination inhibits or delays the onset of castration-resistant prostate cancer in a patient.

Some embodiments are directed to a method of identifying a compound that act as a growth factor for androgen-dependent prostate cancer cells in the absence of dihydrotestosterone, comprising: reducing dihydrotestosterone levels in androgen-dependent prostate cancer cells; administering one or more compounds to the androgen-dependent prostate cancer cells; and measuring cell cycle entry or a specific phase of the cell cycle in the androgen-dependent prostate cancer cells, wherein an increase in cell cycle entry or a specific phase of the cell cycle relative to control cells indicates that the compound acts as a growth factor.

In some embodiments, the androgen-dependent prostate cancer cells are in cell culture or a pseudo-orthotopic chamber mouse model.

In some embodiments, the method further comprises identifying compounds that are both a growth factor and cytotoxic to androgen-dependent prostate cancer cells by screening compounds that are growth factors in a cellular cytotoxicity assay.

In some embodiments, the cellular cytotoxicity assay monitors apoptosis of cells having increased cell cycle entry or increased mitosis.

In some embodiments, the compounds are screened for the modulation of tubulin polymerization.

In some embodiments, cell cycle entry is measured by flow cytometry.

In some embodiments, an inhibitor of cytochrome P450-17 (CYP17), a 5α-reducase inhibitor, or both are administered to reduce the dihydrotestosterone levels. In some embodiments, a selective inhibitor of the 17,20-lyase activity of cytochrome P450-17 (CYP17), a 5α-reducase inhibitor, or both are administered to reduce the dihydrotestosterone levels. In some embodiments, a selective inhibitor of the 17,20-lyase activity of cytochrome P450-17 (CYP17) is administered to reduce the dihydrotestosterone levels. In some embodiments, a 5α-reducase inhibitor is administered to reduce the dihydrotestosterone levels. In some embodiments, serum is removed from the cells to reduce dihydrotestosterone levels.

Some embodiments are directed to a method of identifying a compound that induces androgen-dependent prostate cancer cells to enter cell cycle in the absence of dihydrotestosterone, comprising: reducing the amount of dihydrotestosterone that is in contact with a population of androgen-dependent prostate cancer cells; contacting said population of androgen-dependent prostate cancer cells with a candidate compound; and determining or measuring whether said population of androgen-dependent prostate cancer cells enters cell cycle, a specific phase of the cell cycle, or mitosis after contact with said candidate compound, wherein said compound is identified as one that induces androgen-dependent prostate cancer cells to enter cell cycle in the absence of dihydrotestosterone when said population of androgen-dependent prostate cancer cells enters cell cycle, a specific phase of the cell cycle, or mitosis after contact with said candidate compound. In some embodiments, the androgen-dependent prostate cancer cells are in cell culture or a pseudo-orthotopic chamber mouse model. In some embodiments, the method further comprises determining whether said compound that induces androgen-dependent prostate cancer cells to enter cell cycle in the absence of dihydrotestosterone is cytotoxic to said androgen-dependent prostate cancer cells. In some embodiments, a cellular cytotoxicity assay is used to determine if said identified compound is cytotoxic to said androgen-dependent prostate cancer cells. In some embodiments, the cytotoxicity assay evaluates tubulin polymerization. In some embodiments, cell cycle entry, a specific phase of cell cycle, or mitosis is evaluated using flow cytometry. In some embodiments, an inhibitor of cytochrome P450-17 (CYP17), a 5α-reducase inhibitor, or both are administered to reduce the dihydrotestosterone levels. In some embodiments, a selective inhibitor of the 17,20-lyase activity of cytochrome P450-17 (CYP17), a 5α-reducase inhibitor, or both are administered to reduce the dihydrotestosterone levels. In some embodiments, a selective inhibitor of the 17,20-lyase activity of cytochrome P450-17 (CYP17) is administered to reduce the dihydrotestosterone levels. In some embodiments, a 5α-reducase inhibitor is administered to reduce the dihydrotestosterone levels. In some embodiments, a hormonal therapy agent selected from the group consisting of deslorelin; nafarelin; cetrorelix; and ganirelix is administered to reduce the dihydrotestosterone levels. In some embodiments, serum is removed from the cells to reduce dihydrotestosterone levels. In some embodiments, said candidate compound is a compound of Formula (I), or a pharmaceutically acceptable salt of Formula (I): 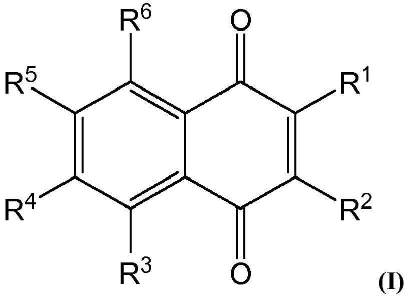 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl. In some embodiments, R¹, R², R³, R⁴, R⁵ and R⁶ are each hydrogen. In some embodiments, R¹ is methyl; R³ is -OH; and R², R⁴, R⁵ and R⁶ are each hydrogen. In some embodiments, R³ and R⁶ are each -OH; and R¹, R², R⁴ and R⁵ are each hydrogen. In some embodiments, R³ is -OH; and R¹, R², R⁴, R⁵ and R⁶ are each hydrogen. In some embodiments, R¹ and R² are each -SCH₂CH₂OH; and R³, R⁴, R⁵ and R⁶ are each hydrogen. In some embodiments, R¹ and R² are each -OCH₃; and R³, R⁴, R⁵ and R⁶ are each hydrogen. In some embodiments, R¹ is -OCH₃; and R², R³, R⁴, R⁵ and R⁶ are each hydrogen. In some embodiments, R¹ is methyl; and R², R³, R⁴, R⁵ and R⁶ are each hydrogen. In some embodiments, R¹ and R² are each chloro; and R³, R⁴, R⁵ and R⁶ are each hydrogen. In some embodiments, R¹ is -OH; and R², R³, R⁴, R⁵ and R⁶ are each hydrogen. In some embodiments, R¹ is phytenyl; R² is methyl; and R³, R⁴, R⁵ and R⁶ are each hydrogen. In some embodiments, R¹ and R⁴ are each t-butyl; and R², R³, R⁵ and R⁶ are each hydrogen. In some embodiments, R¹ is -OH; R² is -CH₂-CH=C(CH₃)₂; and R³, R⁴, R⁵ and R⁶ are each hydrogen. In some embodiments, the compound of Formula (I) is a selected from among the group consisting of: the compound where R¹, R², R³, R⁴, R⁵ and R⁶ are each hydrogen; the compound where R³ and R⁶ are each -OH; and R¹, R², R⁴ and R⁵ are each hydrogen; the compound where R³ is -OH; and R¹, R², R⁴, R⁵ and R⁶ are each hydrogen; the compound where R¹ and R² are each -SCH₂CH₂OH; and R³, R⁴, R⁵ and R⁶ are each hydrogen; the compound where R¹ and R² are each -OCH₃; and R³, R⁴, R⁵ and R⁶ are each hydrogen; the compound where R¹ is -OCH₃; and R², R³, R⁴, R⁵ and R⁶ are each hydrogen; the compound where R¹ is methyl; and R², R³, R⁴, R⁵ and R⁶ are each hydrogen; the compound where R¹ and R² are each chloro; and R³, R⁴, R⁵ and R⁶ are each hydrogen; the compound where R¹ is -OH; and R², R³, R⁴, R⁵ and R⁶ are each hydrogen; the compound where R¹ is phytenyl; R² is methyl; and R³, R⁴, R⁵ and R⁶ are each hydrogen; the compound where R¹ and R⁴ are each t-butyl; and R², R³, R⁵ and R⁶ are each hydrogen; and the compound where R¹ is -OH; R² is -CH₂-CH=C(CH₃)₂; and R³, R⁴, R⁵ and R⁶ are each hydrogen.

Experiments described herein also concern the discovery that the combination of Casodex and plumbagin and/or Orteronel (also known as Tak-700) and plumbagin are effective prostate cancer therapeutics. Unexpectedly, it was found that the combination of Orteronel and plumbagin reduced prostate tumor size significantly and more efficiently than the combination of Casodex and plumbagin. In these experiments, platinum chambers were placed in the dorsal skinfold of nude mice by surgery. Two days later, minced prostate tissue from BalbC mice (syngeneic) was grafted into the chambers and allowed to vascularize for 7 to 10 days. Small tumor cell spheroids were implanted into each chamber. PTEN-P2 stably transfected with H2B-GFP fusion protein (PTEN-P2/GFP) cells were used in these experiments. When tumor vascularization was established (about 5-7 days), the animals were either (A) surgically castrated to inhibit androgen production; or (B) allowed to naturally produce androgens. The mice were then treated with drug combinations (including pre-treatment and concurrent treatment to study the effect of timing for various combinations). In some experiments, test compound treatment was 1 mg/kg (DMSO and PEG30%) via intraperitoneal injection, once/day.

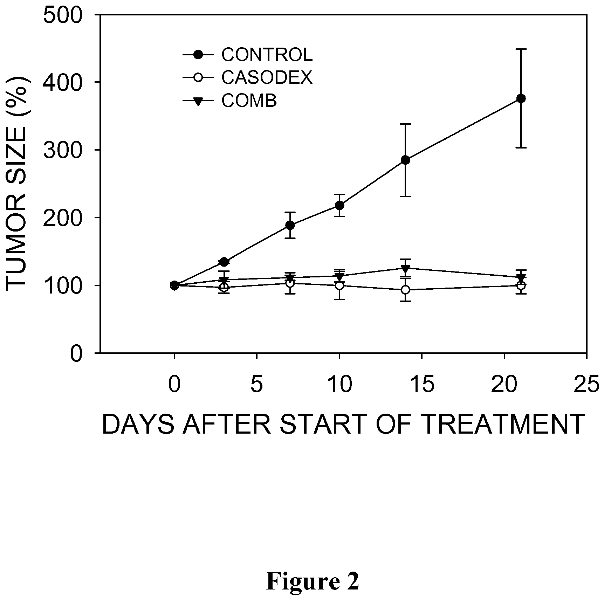

The above protocol was followed with PTEN-P2 tumor spheroids implanted in dorsal skinfold chambers in nude mice, with either no treatment (control), treatment with bicalutamide (i.e. Casodex) at 10 mg/kg po, once/day, and treatment with bicalutamide (i.e. Casodex) at 10 mg/kg po, once/day in combination with plumbagin 1 mg/kg ip once/day. The results of this experiment are summarized in Figure 2, comparing tumor growth without treatment ("CONTROL"), with Casodex ("CASODEX"), and with plumbagin and Casodex ("COMB"). Figure 2 illustrates that treatment with bicalutamide and a combination treatment with bicalutamide and plumbagin drastically reduces tumor size. Unexpectedly, the combination treatment appears, at first glance, equal to or slightly less effective than treatment with bicalutamide alone. Without wishing to be bound to a particular theory, it is known that bicalutamide slows tumor growth, rather than induces apoptosis of tumor cells. Schweizer et al., Therapeutic Advances in Urology, 4(4), 167-178. As we have discovered, plumbagin induces both mitosis and apoptosis (via several proposed mechanisms). Plumbagin also recognizes the colchicine binding site of tubulin and inhibits in vitro tubulin polymerization. *See* Acharya et al., Biochemistry 2008, 47(3), 7838-45. Given these effects, and in view of plumbagin's other proposed apoptotic mechanisms, compounds that slow tumor growth, in particular compounds that bind to or interact with the andorgen receptor, may counteract one or more of plumbagin's apoptotic effects, thereby resulting in a less efficient treatment.

Using the above-described protocol individual compounds of Formula (I) (i.e., plumbagin) can be (A) examined at various concentrations; (B) examined at various concentrations with the administration (pre-treatment, concurrent, or post-treatment) of a selective inhibitor of 17,20-lyase activity of cytochrome P450-17 (CYP17) (i.e, orteronel or VT-464; (C) examined at various concentrations with the administration (pre-treatment, concurrent, or post-treatment) of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl}-3-oxo-4-azaandrost-1-ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18β-glycerrhetinic acid; altraric acid; N-butylbenzene-sulfonamide; 3,3'-diindolylmethane; deslorelin; nafarelin; cetrorelix; or ganirelix; and (D) examined at various concentrations with the administration (pre-treatment, concurrent, or post-treatment) of a selective androgen receptor antagonist, an inhibitor of androgen synthesis, a 5α-reductase inhibitor, or a plant-derived inhibitor. Optionally, comparative studies are run against one or more of plumbagin in combination with cyproterone acetate, abiraterone, finasteride, flutamide, nilutamide, ethylstilbestrol (DES), megestrol acetate, fosfestrol, estamustine phosphate, leuprolide, triptorelin, goserelin, histrelin, buserelin, abarelix and/or degarelix.

In more experiments, plumbagin was formulated as an inclusion complex with heptakis(2,6-di-O-methyl)-beta-cyclodextrin. A super-saturated solution of plumbagin in 0.1M of heptakis(2,6-di-O-methyl)-beta-cyclodextrin was stirred for 72 hours at 25+/-0.5 °C and the mixture was filtered through a 0.2 µm nylon membrane filter. The concentration of solubilized plumbagin has been determined by UV-VIS spectroscopy (Amax(263 nm and 417 nm)). Phase-solubility diagram analysis showed K1:1 = 485 M⁻¹.

TAK-700 (Orteronel) was formulated as an inclusion complex with sulfobutyl ether-beta-cyclodextrin, as well. In brief, 100 mg of TAK-700 was suspended and stirred in 19.5 ml of 50 mM solution of sulfobutyl ether-beta-cyclodextrin for 24 h at 25°C. Then the mixture was filtered through a 0.2 µm nylon membrane filter and the concentration of solubilized TAK-700 was determined by UV-VIS spectroscopy (Amax(281 nm)).

The above protocol was followed with PTEN-P2 tumor spheroids implanted in dorsal skinfold chambers in nude mice, with either no treatment ("CONTROL"), treatment with the plumbagin formulation at 1 mg/kg po, once/day ("PLUMB"), treatment with the TAK-700 formulation at 6 mg/kg po, twice/day ("TAK 700 CD"), treatment with a combination of the TAK-700 formulation at 6 mg/kg po, twice/day and the plumbagin formulation at 1 mg/kg po, once/day ("TAK 700 CD + PLUMB"), treatment with castration ("CAST"), and treatment with castration and the plumbagin formulation at 1 mg/kg po, once/day ("CAST + PLUMB"). The results of this experiment demonstrated that the combination of TAK-700 and plumbagin drastically and unexpectedly reduced tumor size and considerably more effectively reduced tumor size as compared to the combination of Casodex and plumbagin.

Accordingly, some embodiments concern methods of reducing the size of a prostate cancer tumor in a subject, comprising selecting a subject (e.g., a mammal or a human) to receive a therapy that reduces the size of a prostate cancer tumor and providing to said selected subject orteronel and plumbagin. In some embodiments, the method further includes determining or measuring a reduction in prostate cancer tumor size after providing the orteronel and/or plumbagin. The orteronel and plumbagin can be provided separately or can be provided in a mixture and, preferably, said orteronel and/or said plumabagin or both are formulated with beta-cyclodextrin. Thus, some embodiments concern compositions that comprise orteronel and plumbagin and preferably, in said compositions the orteronel and/or plumbagin or both are formulated with beta-cyclodextrin. These compositions may include for example, injectable formulations, pills, tablets, and gel-caps, syrups, or elixirs. The compositions that comprise orteronel and plumbagin can be for use in reducing the size of a prostate cancer tumor in a subject. Some embodiments then include use of orteronel and plumbagin for reducing the size of a prostate cancer tumor in a subject.

### BRIEF DESCRIPTION OF THE DRAWINGS

FIGURE 1 illustrates the steroid/androgen synthesis pathway.
FIGURE 2 compares the growth of tumors without treatment ("CONTROL"), with Casodex ("CASODEX"), and with plumbagin and Casodex ("COMB").
FIGURE 3 shows the effect of plumbagin on cellular mitosis (MI) and apoptosis (AP) at various concentrations.
FIGURE 4 compares the growth of tumors with a cyclodextrin formulation of TAK-700 ("TAK 700 CD"), with plumbagin ("PLUMB"), with a cyclodextrin formulation of TAK-700 and plumbagin ("TAK 700 CD + PLUMB"), with castration and plumbagin ("CAST + PLUMB"), with castration ("CAST"), and without treatment ("CONTROL").

### DETAILED DESCRIPTION

### I. Definitions

Unless defined otherwise, all technical and scientific terms used herein have the same meaning as is commonly understood by one of ordinary skill in the art. In the event that there are a plurality of definitions for a term herein, those in this section prevail unless stated otherwise.

As used herein, any "R" group(s) such as, without limitation, R, R¹, R², R³, R⁴, R⁵, R⁶, R⁷, R⁸, R⁹, R¹⁰, R¹¹, R¹², R¹³, and R¹⁴ represent substituents that can be attached to the indicated atom. An R group may be substituted or unsubstituted.

As used herein, "Cₐ to C_{b}" in which "a" and "b" are integers refer to the number of carbon atoms in an alkyl, alkenyl or alkynyl group, or the number of carbon atoms in the ring of a cycloalkyl, cycloalkenyl, cycloalkynyl, aryl, heteroaryl or heteroalicyclyl group. That is, the alkyl, alkenyl, alkynyl, ring of the cycloalkyl, ring of the cycloalkenyl, ring of the cycloalkynyl, ring of the aryl, ring of the heteroaryl or ring of the heteroalicyclyl can contain from "a" to "b", inclusive, carbon atoms. Thus, for example, a "C₁ to C₄ alkyl" group refers to all alkyl groups having from 1 to 4 carbons, that is, CH₃-, CH₃CH₂-, CH₃CH₂CH₂-, (CH₃)₂CH-, CH₃CH₂CH₂CH₂-, CH₃CH₂CH(CH₃)- and (CH₃)₃C-. If no "a" and "b" are designated with regard to an alkyl or alkenyl group, the broadest range described in these definitions is to be assumed.

As used herein, "alkyl" refers to a straight or branched hydrocarbon chain that comprises a fully saturated (no double or triple bonds) hydrocarbon group. The alkyl group may have 1 to 20 carbon atoms (whenever it appears herein, a numerical range such as "1 to 20" refers to each integer in the given range; *e.g.,* "1 to 20 carbon atoms" means that the alkyl group may consist of 1 carbon atom, 2 carbon atoms, 3 carbon atoms, *etc.,* up to and including 20 carbon atoms, although the present definition also covers the occurrence of the term "alkyl" where no numerical range is designated). The alkyl group may also be a medium size alkyl having 1 to 10 carbon atoms. The alkyl group could also be a lower alkyl having 1 to 6 carbon atoms. The alkyl group of the compounds may be designated as "C₁-C₄ alkyl" or similar designations. By way of example only, "C₁-C₄ alkyl" indicates that there are one to four carbon atoms in the alkyl chain, i.e., the alkyl chain is selected from methyl, ethyl, propyl, iso-propyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl and hexyl. The alkyl group may be substituted or unsubstituted.

As used herein, "alkenyl" refers to an alkyl group that contains in the straight or branched hydrocarbon chain one or more double bonds. An alkenyl group may be unsubstituted or substituted.

The term "halogen" as used herein, means any one of the radio-stable atoms of column 7 of the Periodic Table of the Elements, such as, fluorine, chlorine, bromine and iodine.

Whenever a group is described as being "optionally substituted" that group may be unsubstituted or substituted with one or more of the indicated substituents. Likewise, when a group is described as being "unsubstituted or substituted" if substituted, the substituent may be selected from one or more the indicated substituents. If no substituents are indicated, it is meant that the indicated "optionally substituted" or "substituted" group may be substituted with one or more group(s) individually and independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxyl, alkoxy, aryloxy, acyl, mercapto, alkylthio, arylthio, cyano, halogen, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalomethanesulfonamido, amino, mono-substituted amino group and di-substituted amino group, and protected derivatives thereof.

The term "naphthoquinone analog" refers to a compound of Formula (I) wherein R¹, R², R³, R⁴, R⁵, and R⁶ are as defined herein.

The term "pharmaceutically acceptable salt" refers to a salt of a compound that does not cause significant irritation to an organism to which it is administered and does not abrogate the biological activity and properties of the compound. In some embodiments, the salt is an acid addition salt of the compound. Pharmaceutical salts can be obtained by reacting a compound with inorganic acids such as hydrohalic acid (e.g., hydrochloric acid or hydrobromic acid), sulfuric acid, nitric acid and phosphoric acid. Pharmaceutical salts can also be obtained by reacting a compound with an organic acid such as aliphatic or aromatic carboxylic or sulfonic acids, for example formic, acetic, succinic, lactic, malic, tartaric, citric, ascorbic, nicotinic, methanesulfonic, ethanesulfonic, p-toluensulfonic, salicylic or naphthalenesulfonic acid. Pharmaceutical salts can also be obtained by reacting a compound with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glucamine, tris(hydroxymethyl)methylamine, C₁-C₇ alkylamine, cyclohexylamine, triethanolamine, ethylenediamine, and salts with amino acids such as arginine and lysine.

It is understood that, in any compound described herein having one or more chiral centers, if an absolute stereochemistry is not expressly indicated, then each center may independently be of R-configuration or S-configuration or a mixture thereof. Thus, the compounds provided herein may be disatereomerically pure, disatereomerically enriched, or may be stereoisomeric mixtures. In addition it is understood that, in any compound described herein having one or more double bond(s) generating geometrical isomers that can be defined as E or Z, each double bond may independently be E or Z a mixture thereof. Likewise, it is understood that, in any compound described, all tautomeric forms are also intended to be included.

The term "pharmaceutical composition" refers to a mixture of a compound disclosed herein with other chemical components, such as diluents or carriers. The pharmaceutical composition facilitates administration of the compound to an organism. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid and salicylic acid. Pharmaceutical compositions will generally be tailored to the specific intended route of administration.

The term "physiologically acceptable" defines a carrier, diluent or excipient that does not abrogate the biological activity and properties of the compound.

As used herein, a "carrier" refers to a compound that facilitates the incorporation of a compound into cells or tissues. For example, without limitation, dimethyl sulfoxide (DMSO) is a commonly utilized carrier that facilitates the uptake of many organic compounds into cells or tissues of a subject.

As used herein, a "diluent" refers to an ingredient in a pharmaceutical composition that lacks pharmacological activity but may be pharmaceutically necessary or desirable. For example, a diluent may be used to increase the bulk of a potent drug whose mass is too small for manufacture and/or administration. It may also be a liquid for the dissolution of a drug to be administered by injection, ingestion or inhalation. A common form of diluent in the art is a buffered aqueous solution such as, without limitation, phosphate buffered saline that mimics the composition of human blood.

As used herein, an "excipient" refers to an inert substance that is added to a pharmaceutical composition to provide, without limitation, bulk, consistency, stability, binding ability, lubrication, disintegrating ability etc., to the composition. A "diluent" is a type of excipient.

As used herein, a "subject" refers to an animal that is the object of treatment, observation or experiment. "Animal" includes cold- and warm-blooded vertebrates and invertebrates such as fish, shellfish, reptiles and, in particular, mammals. "Mammal" includes, without limitation, mice, rats, rabbits, guinea pigs, dogs, cats, sheep, goats, cows, horses, primates, such as monkeys, chimpanzees, and apes, and, in particular, humans. In some embodiments, the subject is human.

As used herein, the terms "treating," "treatment," "therapeutic," or "therapy" do not necessarily mean total cure or abolition of the disease or condition. Any alleviation of any undesired signs or symptoms of a disease or condition, to any extent can be considered treatment and/or therapy. Furthermore, treatment may include acts that may worsen the patient's overall feeling of well-being or appearance.

The term "therapeutically effective amount" is used to indicate an amount of an active compound, or pharmaceutical agent, that elicits the biological or medicinal response indicated. For example, a therapeutically effective amount of compound can be the amount needed to prevent, alleviate or ameliorate symptoms of disease or prolong the survival of the subject being treated. This response may occur in a tissue, system, animal or human and includes alleviation of the signs or symptoms of the disease being treated. Determination of a therapeutically effective amount is well within the capability of those skilled in the art, in view of the disclosure provided herein. The therapeutically effective amount of the compounds disclosed herein required as a dose will depend on the route of administration, the type of animal, including human, being treated, and the physical characteristics of the specific animal under consideration. The dose can be tailored to achieve a desired effect, but will depend on such factors as weight, diet, concurrent medication and other factors which those skilled in the medical arts will recognize.

As used herein, the term "hormone therapy agent" refers to enzulatomide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl-5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); VT-464; orteronel; aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl}-3-oxo-4-azaandrost-1-ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18B-glycerrhetinic acid; altraric acid; N-butylbenzene-sulfonamide; and 3,3'-diindolylmethane. Some hormone therapy agents are compounds that inhibit the synthesis and/or conversion of testosterone, such as orteronel ("testosterone synthesis inhibitors"); whereas, other hormone therapy agents bind to the androgen receptor and thereby inhibit the binding of testosterone to the androgen receptor, such as Casodex ("androgen receptor inhibitor"). Some hormone therapy agents are compounds that both inhibit the synthesis and/or conversion of testosterone and bind to the Androgen receptor, such as arbiraterone ("combined testosterone synthesis and androgen receptor inhibitors").

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least." When used in the context of a process, the term "comprising" means that the process includes at least the recited steps, but may include additional steps. When used in the context of a compound, composition or device, the term "comprising" means that the compound, composition or device includes at least the recited features or components, but may also include additional features or components. The section below describes some of the compounds that can be used to treat cancer, or inhibit or delay the growth of cancer cells, especially prostate cancer cells alone or in combination with one or more androgen deprivation therapies (e.g., castration, hormonal castration, hormonal ablation, or hormone therapy).

### II. Prostate Cancer

In the initial stages, prostate tumor growth is androgen dependent. Androgens are used by prostate cancer cells for both proliferation as well as regulation, are vital for maintaining the growth and survival of the cancer cell. The main androgen that circulates is testosterone, which is mainly produced in the testes. Extragonadal sources of androgen synthesis do, however, exist and may play a role in the development of castration-resistant forms of prostate cancer. Generally, androgen dependent prostate cancer therapy focuses on minimizing testicular synthesis of androgens with luteinizing hormone releasing hormone ("LHRH") agonists or antagonists. Some therapies also focus on modulating the androgen receptor itself, or its downstream signaling pathway.

Androgen dependent prostate cancer will eventually progress into castration-resistant prostate cancer ("CRPC"). Although these patients are "androgen insensitive," researchers have discovered that androgen-responsive genes are still expressed, implying that the androgen-receptor signaling pathway may still be an important target in CRPC patients. Schweizer et al., Therapeutic Advances in Urology, 4(4), 167-178.

There were an estimated 192,280 new cases of prostate cancer diagnosed in the U.S. in 2009 and an estimated 27,360 deaths. About 90% of patients with advanced disease will develop bone metastases, associated with severe pain, loss of mobility, and spinal cord compression. Other affected organs may include the liver, lungs and brain. Advanced prostate cancer is resistant to hormone therapy, radiation and conventional chemotherapy. Although the 5-year survival rate is close to 100% for local disease, it drops to 30% for advanced cancer.

There have been some advances in the treatment of prostate cancer recently, including new surgical approaches and improvements in radiotherapy. For example:
1) In 1986, surgeons developed a technique (using *da Vinci* Prostatectomy) that allowed the removal of the prostate while minimizing nerve damage, thereby decreasing adverse side effects.
2) In addition, clinical researchers improved a long-established radiotherapy technique known as brachytherapy, which involves the implantation of a small amount of radioactive material (seeds) into the prostate. This radiation therapy method is an effective treatment for early-stage prostate cancer.
3) There have also been advances in hormonal therapy for prostate cancer including the development of gonadotropin-releasing hormone (GnRH) agonists, which inhibit the ability of the pituitary gland to stimulate the testes to make testosterone.
4) Advances have also been made in chemotherapy for prostate cancer. In 2004, results from two large NCI-sponsored clinical trials showed that use of the drug docetaxel could prolong the survival of men who had advanced prostate cancer which no longer responded to hormonal therapy.

Unfortunately, should the prostate-specific antigen (PSA) level remain above zero after radical prostatectomy is performed, with conventional therapy or with advanced therapy using da Vinci Prostatectomy, this indicates that the prostate cancer has spread outside the capsule, i.e., disseminated disease, and to date, there is no curable treatment for this.

Thus, all current hormonal, as well as, chemotherapy treatment regimens for such disseminated androgen dependent prostate cancers are palliative. Subsequently, even if there have been advances in the treatment of prostate cancer, finding new strategies for treatment of disseminated disease remains a crucial challenge. The section below provides more details on the use of compounds of Formula (I) to inhibit or delay the growth of cancer cells, in particular prostate cancer cells.

### II. Compounds of Formula (I)

Some embodiments disclosed herein relate to a compound of Formula (I), or a pharmaceutically acceptable salt thereof, and methods of using these compounds with a hormone therapy agent, as described herein, to inhibit, delay, treat, or prevent prostate cancer cell growth or prostate cancer in a subject in need thereof. Other embodiments disclosed herein relate to the use of a compound of Formula (I), or a pharmaceutically acceptable salt thereof, with a hormone therapy agent, as described herein, to inhibit, delay, treat, or prevent prostate cancer cell growth or prostate cancer in a subject in need thereof. Other embodiments disclosed herein relate to the use of a compound of Formula (I), or a pharmaceutically acceptable salt thereof, with a hormone therapy agent, as described herein, in the manufacture of a medicament for inhibiting, delaying, treating, or preventing prostate cancer cell growth or prostate cancer in a subject in need thereof. Other embodiments, disclosed herein relate to a product combination and/or the use of a product combination containing a compound of Formula (I), or a pharmaceutically acceptable salt thereof, and a hormone therapy agent, as described herein, to inhibit, delay, treat, or prevent prostate cancer cell growth or prostate cancer in a subject in need thereof and/or for the manufacture of a product combination for inhibiting, delaying, treating, or preventing prostate cancer cell growth or prostate cancer in a subject in need thereof.

In some embodiments, the compound of Formula (I) has the following structure: 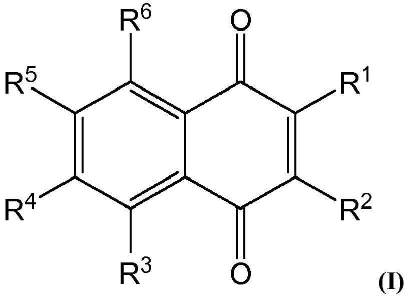 wherein: R¹ can be selected from hydrogen, halogen, an optionally substituted C₁₋₁₈ alkyl, an optionally substituted C₂₋₁₈ alkenyl, -OR⁷ and -SR⁸; R² can be selected from hydrogen, halogen, an optionally substituted C₁₋₆ alkyl, an optionally substituted C₂₋₆ alkenyl, -OR⁹ and -SR¹⁰; R³ can be selected from hydrogen, an optionally substituted C₁₋₆ alkyl, and - OR¹¹; R⁴ can be selected from hydrogen, an optionally substituted C₁₋₆ alkyl, and -OR¹²; R⁵ can be selected from hydrogen, an optionally substituted C₁₋₆ alkyl, and -OR¹³; R⁶ can be selected from hydrogen, an optionally substituted C₁₋₆ alkyl, and -OR¹⁴; and R⁷, R⁸, R⁹, R¹⁰, R¹¹, R¹², R¹³, and R¹⁴ can be independently selected from hydrogen and an optionally substituted C₁₋₆ alkyl.

In some embodiments, R¹ can be hydrogen. In some embodiments, R¹ can be halogen. In some embodiments, R¹ can be chloro. In some embodiments, R¹ can be an optionally substituted C₁₋₁₈ alkyl. Examples of optionally substituted C₁₋₁₈-alkyls include, but are not limited to, optionally substituted variants of the following: methyl, ethyl, n-propyl, isopropyl, n-butyl, isobutyl, tert-butyl, pentyl, hexyl, heptyl, octyl, nonanyl, decanyl, undecanyl, dodecanyl, tridecanyl, tetradecanyl, pentadecanyl, hexadecanyl, heptadecanyl, octadecanyl, and phytanyl. Optionally substituted C₁₋₁₈-alkyls can be branched or straight-chained. In some embodiments, R¹ can be an optionally substituted C₁₋₆ alkyl. In some embodiments, R¹ can be methyl. In some embodiments, R¹ can be t-butyl. In some embodiments, R¹ can be an optionally substituted C₂₋₁₈ alkenyl. Examples of optionally substituted C₂₋₁₈-alkenyls include, but are not limited to, optionally substituted variants of the following: ethenyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, and phytenyl. Optionally substituted C₂₋₁₈-alkenyls can be branched or straight-chained, and can include one or more double bonds. In some embodiments, R¹ can be an optionally substituted C₂₋₆ alkenyl. In some embodiments, R¹ can be -OR⁷, wherein R⁷ is hydrogen. In some embodiments, R¹ can be -OR⁷, wherein R⁷ is an optionally substituted C₁₋₆ alkyl. In some embodiments, R¹ can be -OR⁷, wherein R⁷ is methyl. In some embodiments, R¹ can be -SR⁸, wherein R⁸ is hydrogen. In some embodiments, R¹ can be -SR⁸, wherein R⁸ is an optionally substituted C₁₋₆ alkyl. In some embodiments, R¹ can be -SR⁸, wherein R⁸ is C₁₋₆ alkyl optionally substituted with hydroxy. In some embodiments, R¹ can be -SR⁸, wherein R⁸ is - CH₂CH₂OH.

In some embodiments, R² can be hydrogen. In some embodiments, R² can be halogen. In some embodiments, R² can be chloro. In some embodiments, R² can be an optionally substituted C₁₋₆ alkyl. Examples of optionally substituted C₁₋₆-alkyls include optionally substituted variants of the following: methyl, ethyl, n-propyl, isopropyl, n-butyl, isobutyl, tert-butyl, pentyl (branched and straight-chained), and hexyl (branched and straight-chained). In some embodiments, R² can be methyl. In some embodiments, R² can be an optionally substituted C₂₋₆ alkenyl. Examples of optionally substituted C₂₋₆-alkenyls include optionally substituted variants of the following: ethenyl, propenyl, butenyl, pentenyl (branched and straight-chained), and hexenyl (branched and straight-chained). In some embodiments, R² can be -CH₂-CH=C(CH₃)₂. In some embodiments, R² can be -OR⁹, wherein R⁹ is hydrogen. In some embodiments, R² can be -OR⁹, wherein R⁹ is an optionally substituted C₁₋₆ alkyl. In some embodiments, R² can be -OR⁹, wherein R⁹ is methyl. In some embodiments, R² can be -SR¹⁰, wherein R¹⁰ is hydrogen. In some embodiments, R² can be -SR¹⁰, wherein R¹⁰ is an optionally substituted C₁₋₆ alkyl. In some embodiments, R² can be -SR¹⁰, wherein R¹⁰ is C₁₋₆ alkyl optionally substituted with hydroxy. In some embodiments, R² can be -SR¹⁰, wherein R¹⁰ is -CH₂CH₂OH.

In some embodiments, R³ can be hydrogen. In some embodiments, R³ can be an optionally substituted C₁₋₆ alkyl. In some embodiments, R³ can be -OR¹¹, wherein R¹¹ is hydrogen. In some embodiments, R³ can be -OR¹¹, wherein R¹¹ is an optionally substituted C₁₋₆ alkyl.

In some embodiments, R⁴ can be hydrogen. In some embodiments, R⁴ can be an optionally substituted C₁₋₆ alkyl. In some embodiments, R⁴ can be t-butyl. In some embodiments, R⁴ can be -OR¹², wherein R¹² is hydrogen. In some embodiments, R⁴ can be -OR¹², wherein R¹² is an optionally substituted C₁₋₆ alkyl.

In some embodiments, R⁵ can be hydrogen. In some embodiments, R⁵ can be an optionally substituted C₁₋₆ alkyl. In some embodiments, R⁵ can be -OR¹³, wherein R¹³ is hydrogen. In some embodiments, R⁵ can be -OR¹³, wherein R¹³ is an optionally substituted C₁₋₆ alkyl.

In some embodiments, R⁶ can be hydrogen. In some embodiments, R⁶ can be an optionally substituted C₁₋₆ alkyl. In some embodiments, R⁶ can be -OR¹⁴, wherein R¹⁴ is hydrogen. In some embodiments, R⁶ can be -OR¹⁴, wherein R¹³ is an optionally substituted C₁₋₆ alkyl.

In some embodiments, R⁷, R⁸, R⁹, R¹⁰, R¹¹, R¹², R¹³, and R¹⁴ can be independently selected from hydrogen. In some embodiments, R⁷, R⁸, R⁹, R¹⁰, R¹¹, R¹², R¹³, and R¹⁴ can be independently selected from C₁₋₆ alkyl. In some embodiments, R⁷, R⁸, R⁹, R¹⁰, R¹¹, R¹², R¹³, and R¹⁴ can be independently selected from C₁₋₆ alkyl, wherein the C₁₋₆ alkyl can be optionally substituted with a group selected from halogen, hydroxy, and C₁₋₄ alkyl.

In some embodiments, R¹ can be selected from hydrogen, halogen, an optionally substituted C₁₋₆ alkyl, -OR⁷ and -SR⁸; R² can be selected from hydrogen, halogen, an optionally substituted C₁₋₆ alkyl, -OR⁹ and -SR¹⁰; R³ can be selected from hydrogen and -OR¹¹; R⁴ can be selected from hydrogen and an optionally substituted C₁₋₆ alkyl; R⁵ can be hydrogen; R⁶ can be selected from hydrogen and -OR¹⁴; and R⁷, R⁸, R⁹, R¹⁰, R¹¹, R¹², R¹³, and R¹⁴ can be independently selected from hydrogen and an optionally substituted C₁₋₆ alkyl.

In some embodiments, R¹, R², R³, R⁴, R⁵ and R⁶ can each be hydrogen. In some embodiments, R¹ can be methyl; R³ can be -OH; and R², R⁴, R⁵ and R⁶ can each be hydrogen. In some embodiments, R³ and R⁶ can each be -OH; and R¹, R², R⁴ and R⁵ can each be hydrogen. In some embodiments, R³ can be -OH; and R¹, R², R⁴, R⁵ and R⁶ can each be hydrogen. In some embodiments, R¹ and R² can each be -SCH₂CH₂OH; and R³, R⁴, R⁵ and R⁶ can each be hydrogen. In some embodiments, R¹ and R² can each be - OCH₃; and R³, R⁴, R⁵ and R⁶ can each be hydrogen. In some embodiments, R¹ can be - OCH₃; and R², R³, R⁴, R⁵ and R⁶ can each be hydrogen. In some embodiments, R¹ can be methyl; and R², R³, R⁴, R⁵ and R⁶ can each be hydrogen. In some embodiments, R¹ and R² can each be chloro; and R³, R⁴, R⁵ and R⁶ can each be hydrogen. In some embodiments, R¹ can be -OH; and R², R³, R⁴, R⁵ and R⁶ can each be hydrogen. In some embodiments, R¹ can be phytenyl; R² can be methyl; and R³, R⁴, R⁵ and R⁶ can each be hydrogen. In some embodiments, R¹ and R⁴ can each be t-butyl; and R², R³, R⁵ and R⁶ can each be hydrogen. In some embodiments, R¹ can be -OH; R² can be -CH₂-CH=C(CH₃)₂; and R³, R⁴, R⁵ and R⁶ can each be hydrogen.

In some embodiments, at least one of R¹, R², R³, R⁴, R⁵ and R⁶ cannot be hydrogen. In some embodiments, when R¹ is methyl; and R², R⁴, R⁵ and R⁶ are each hydrogen; then R³ cannot be -OH. In some embodiments, when R¹, R², R⁴ and R⁵ are each hydrogen; then at least one of R³ and R⁶ cannot be -OH. In some embodiments, when R¹, R², R⁴, R⁵ and R⁶ are each hydrogen; then R³ cannot be -OH. In some embodiments, when R³, R⁴, R⁵ and R⁶ are each hydrogen; then at least one of R¹ and R² cannot be -SCH₂CH₂OH. In some embodiments, when R³, R⁴, R⁵ and R⁶ are each hydrogen; then at least one of R¹ and R² cannot be -OCH₃. In some embodiments, when R², R³, R⁴, R⁵ and R⁶ are each hydrogen; then R¹ cannot be -OCH₃. In some embodiments, when R², R³, R⁴, R⁵ and R⁶ are each hydrogen; then R¹ cannot be methyl. In some embodiments, when R³, R⁴, R⁵ and R⁶ are each hydrogen; then at least one of R¹ and R² cannot be chloro. In some embodiments, when R², R³, R⁴, R⁵ and R⁶ are each hydrogen; then R¹ cannot be -OH. In some embodiments, when R² is methyl; and R³, R⁴, R⁵ and R⁶ are each hydrogen; then R¹ cannot be phytenyl. In some embodiments, when R², R³, R⁵ and R⁶ are each hydrogen; then at least one of R¹ and R⁴ cannot be t-butyl. In some embodiments, when R² is -CH₂-CH=C(CH₃)₂; and R³, R⁴, R⁵ and R⁶ are each hydrogen; then R¹ cannot be -OH.

Examples of compounds of Formula (I) include, but are not limited to the following: 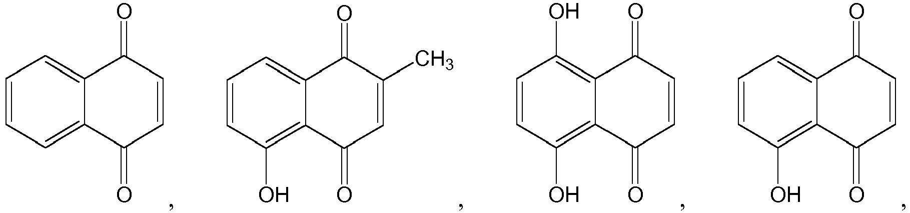 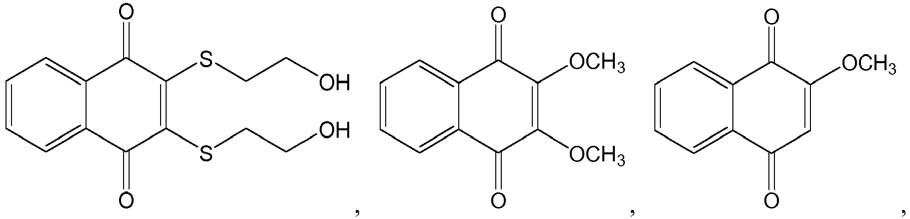 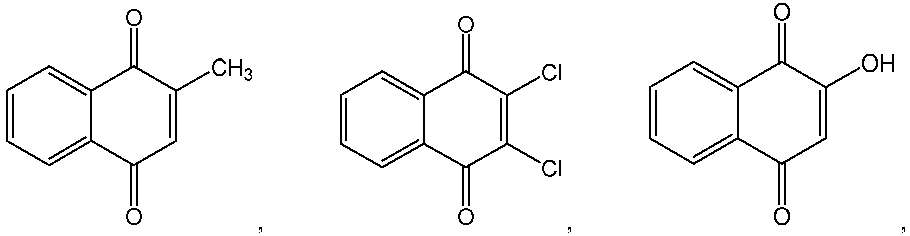 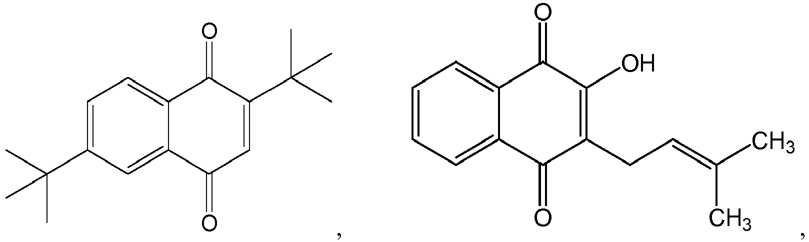 and

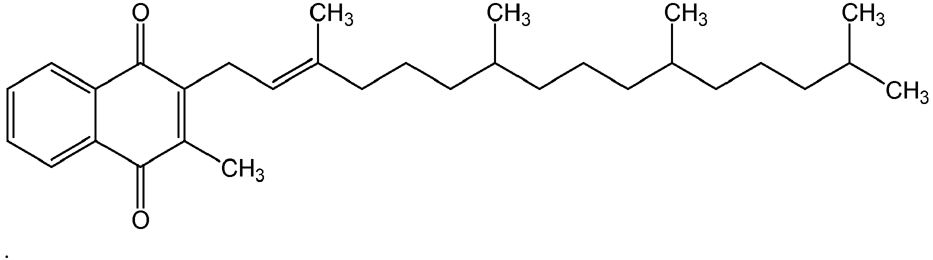

In some embodiments, the compound of Formula (I) can be a dimer, such that one of R¹, R², R³, R⁴, R⁵ or R⁶ has the structure of Formula (I). For example, in some embodiments, the compound of Formula (I) can be Lawsone dimer:

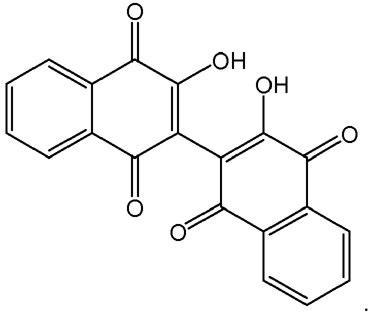

Compounds of Formula (I) have significant anti-cancer properties. For example, plumbagin (5-hydroxy-2-methylnaphthalene-1,4-dione) is a naturally occurring naphthoquinone that can be found in various medicinal herbal species, including *Plumbago zeylanica*, *Statice limonium*, and *Limonium carolinianum.* Plumbagin has demonstrated anticancer effect toward fibrosarcomas (ED₅₀ 0.75 mg/kg body weight) and P388 lymphocytic leukemia (ED₅₀ 4 mg/kg body weight), induced regression of hepatoma, and has inhibited growth and invasion of hormone-refractory prostate cancer. Aziz et al., Cancer Res. 2008, 68(21):9024-322. Furthermore, plumbagin has shown to be a promising chemopreventive/anticarcinogenic agent against intestinal neoplasia.

Without wishing to be bound by theory, it is contemplated that the primary mechanism of cytotoxic action of plumbagin and other quinoid compounds is due to redox-cycling and electrophilic arylation. Plumbagin can be reduced by electron transfer from flavoprotein to a semiquinone radical, which can, in turn, reduce oxygen to superoxide. The resulting superoxide can consequently be converted into hydrogen peroxide, hydroxyl radicals, and/or peroxynitrite, all of which are highly reactive oxygen species (ROS) with potent cytotoxic and tumoricidial effects.

While still not wishing to be bound by theory, an additional antitumor mechanism of plumbagin and related quinones can involve direct arylation of intracellular thiols leading to depletion of glutathione (GSH). Depletion of GSH may ultimately result in alkylation of cellular macromolecules and in their inactivation. Moreover, it has been shown that low dose concentrations of plumbagin (5 umol/L) can inhibit expression of multiple molecular targets, including protein kinase Cq (PKCq), phosphatidylinositol 3-kinase (PI3K), AKT, activation of transcription factors activator protein-1 (AP-1), nuclear factor-κB (NF-κB), and signal transducer and activator of transcription 3 (Stat3) in prostate carcinoma cells. Such activities may contribute to the tumoricidial effects of plumbagin.

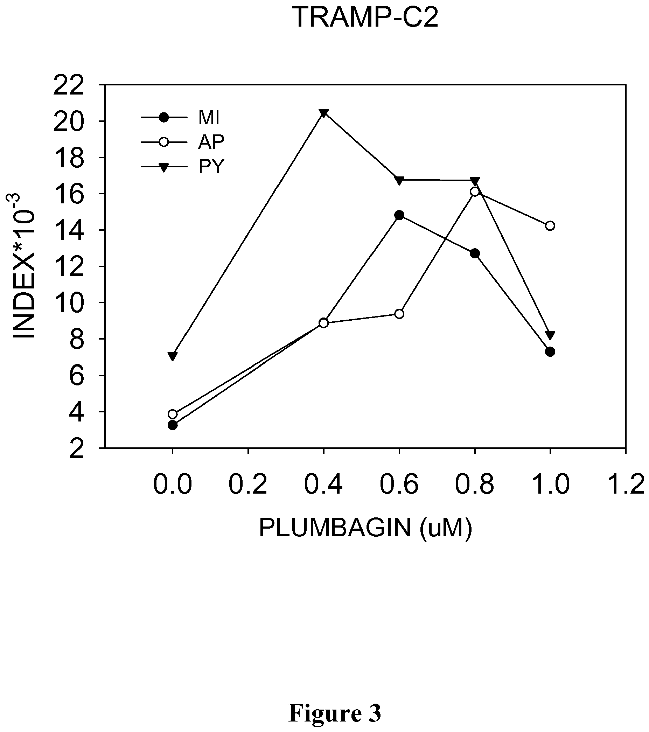

Moreover, while still not wishing to be bound by theory, an additional antitumor mechanism of plumbagin and related quinones can involve inhibition of microtubule polymerization and binding to tubulin. Because one of the defining characteristics of cancer cells is a significantly increased rate of cell cycle entry and/or mitosis, cancer cells are more vulnerable to agents that affect microtubule polymerization than normal cells. Plumbagin recognizes the colchicine binding site of tubulin and also inhibits in vitro tubulin polymerization. *See* Acharya et al., Biochemistry 2008, 47(3), 7838-45. Surprisingly, and described in more detail in the experimental section below, we have also discovered that plumbagin induces cell cycle entry and mitosis in prostate cells (*See* Figure 3).

Studies using plumbagin in pre-clinical models have revealed that treatment with plumbagin can result in slower growth of androgen independent prostate cancer, and that the mechanism behind the slower growth may be due to apoptosis of prostate tumor cells.

It is contemplated that several compounds of Formula (I) have anti-cancer activity and that this anti-cancer activity, especially with respect to prostate cancer, can be significantly improved (e.g., synergy can be obtained) when the compounds are provided in conjunction with certain hormonal therapy agents, described in more detail below. It is believed that plumbagin interacts with the androgen receptor or heat shock proteins that are in communication with the androgen receptor. Accordingly, it is preferred that the plumbagin is provided in combination or in co-administration with a testosterone synthesis inhibitor that does not interact with or bind to the androgen receptor (e.g., a testosterone synthesis inhibitor that does not bind to the androgen receptor, such as orteronel or VT-464).

As mentioned above, although administering to a subject that has cancer (e.g., prostate cancer) one or more compounds of Formula (I) alone or in a combination of compounds of Formula (I) can inhibit the growth of cancerous cells, a significantly improved inhibition of cancer cell growth (e.g., prostate cancer cell growth) can be obtained by providing one or more of the compounds of Formula (I), separately or in a mixture, co-administration, or combination, in conjunction with a hormonal therapy agent that reduces the androgen levels of the patient and/or disrupts androgen receptor signaling (e.g., a testosterone synthesis inhibitor that does not bind to the androgen receptor, such as orteronel or VT-464). That is, some embodiments include methods of inhibiting cancer cell growth (e.g., prostate cancer cell growth or progression of prostate cancer disease) or treating or preventing a cancer (e.g., prostate cancer), wherein a subject having a cancer (e.g., prostate cancer) is identified using conventional clinical or diagnostic techniques (e.g., digital-rectal prostate examination, PSA test, or tissue biopsy); said identified subject is provided one or more compounds of Formula (I) (e.g., plumbagin) and a hormone therapy agent (e.g., a testosterone synthesis inhibitor that does not bind to the androgen receptor, such as orteronel or VT-464). Optionally, the inhibition of cancer (e.g., prostate cancer) or a marker thereof (e.g., PSA) is evaluated after the treatment (e.g., after the combination of plumbagin and the hormone therapy agent is provided). Stated differently, some embodiments of the invention include a combination of one or more of the compounds of Formula (I), formulated for administration separately or together, and a hormonal therapy agent for use in inhibiting or delaying the growth of prostate cancer cells or treating or preventing prostate cancer, wherein, optionally, the hormonal therapy agent is a testosterone synthesis inhibitor, preferably a testosterone synthesis inhibitor that does not bind to the androgen receptor, such as orteronel or VT-464.

### III. Hormone Therapy Agents

Hormone therapy for treating prostate cancer, or inhibiting or delaying prostate cancer cell growth, can also be called androgen deprivation therapy (ADT), chemical castration, or androgen ablation therapy. Androgens can fuel the growth of prostatic cells, including both healthy prostatic cells and cancerous prostatic cells. In some embodiments, a subject suffering from prostate cancer is provided with a hormone therapy agent that reduces the subject's androgen levels.

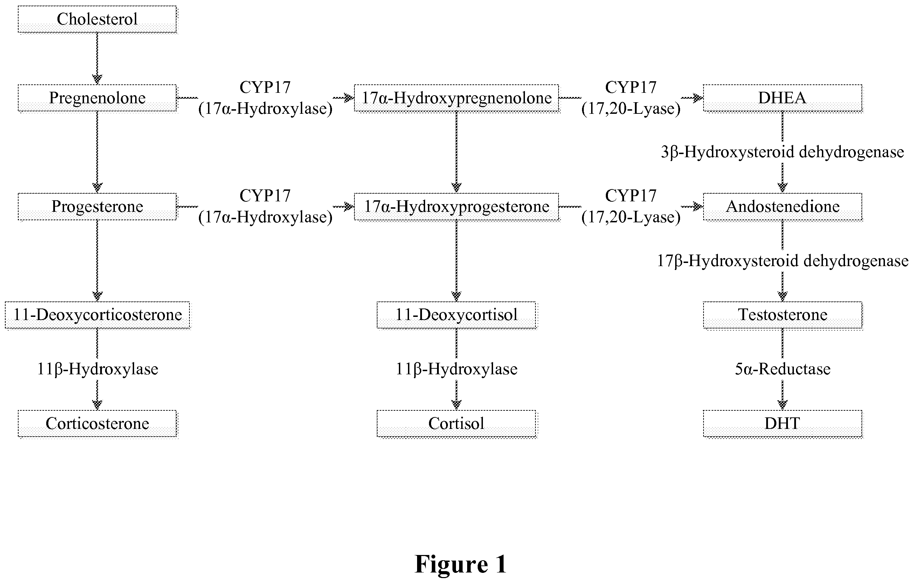

Figure 1 illustrates the steroid/androgen synthesis pathway. In Figure 1, cholesterol is converted to pregnenolone, which then undergoes conversion along the mineralcortioid biosynthesis pathway to progesterone, 11-deoxycorticosterone, and corticosterone (and then to 18-hydroxycorticosterone and aldosterone, not pictured). The conversion to corticosterone occurs via the enzyme 11β-hydroxylase. 11β-hydroxylase is also featured in the glucocorticoid pathway. For the glucocorticoid biosynthesis pathway, pregnenolone or progesterone is converted via the 17α-hydroxylase activity of cytochrome P450-17 ("CYP17") to either 17α-hydroxypregnenolone or 17α-hydroxyprogesterone. 17α-hydroxyprogesterone is converted to 11-deoxycortisol, which in turn is converted to cortisol by 11β-hydroxylase. CYP17 is also featured in the androgen biosynthesis pathway. CYP17, utilizing its 17,20-lyase activity, converts 17α-hydroxypregnenolone to to dehydroepiandrosterone ("DHEA") and 17α-hydroxyprogesterone to adostenedione. Adostenedione, in turn, is converted to testosterone by 17β-hydroxysteroid dehydrogenase, while testosterone is converted to dihydrotestosterone ("DHT") by 5α-reductase.

In some embodiments, a hormonal therapy agent is provided to a patient to selectively inhibit the androgen biosynthesis pathway. Selective inhibition of this pathway is desirable given that a patient receiving such an agent will not require hormone replacement therapy. Hormone replacement therapy is often required when non-selective hormonal therapy agents, such as arbiraterone are provided, resulting in the inhibition of mineralocorticoid biosynthesis and/or glucocorticoid biosynthesis. Such inhibition affords side effects, causes the patient to take additional drugs, can reduce patient compliance, and impair the patient's quality of life. Additionally, it is contemplated that some non-selective hormonal therapy agents, such as arbiraterone, may interfere with or counteract the anti-cancer potential of plumbagin, for example, by competing with plumbagin for binding to the androgen receptor or heat shock proteins associated with the androgen receptor.

In some embodiments, a hormonal therapy agent is provided to a patient to selectively inhibit the 17,20-lyase activity of CYP17. Such inhibition will result in the selective decrease of DHEA and andostenedione production, while not affecting mineralocorticoid biosynthesis and glucocorticoid biosynthesis. Indeed, selectivity targeting CYP17's 17,20-lyase activity, while leaving the 17α-hydroxylase activity of CYP17 relatively undisturbed should afford limited side effects and be less likely to require the concomitant administration of a hormone replacement, such as prednisone.

Inhibitors of 17,20-lyase activity of cytochrome P450-17 ("CYP-17") are known in the art. Steroid-type inhibitors of 17,20-lyase activity are disclosed in, for example, WO 92/15404, WO 93/20097, EP-A 288053, and EP-A 413270. Non-steroid-type compounds are disclosed in, for example, in WO94/27989, WO96/14090, WO97/00257; WO95/09157; U.S. Pat. No. 5,491,161; WO99/18075; WO99/54309; WO03/027085; and EP0724591. Additional compounds include, but are not limited to, compounds disclosed in U.S. Pat. No. 8,236,962; U.S. Pat. No. 8263,635; and U.S. Patent Application No. 20100305078.

Specific examples of selective 17,20-lyase inhibitors for use in certain embodiments include 6-(7-hydroxy-6,7-dihydro-5*H*-pyrrolo[1,2-c]imidazole-7-yl)-2-napthamide; 6-(7-hydroxy-6,7-dihydro-5*H*-pyrrolo[1,2-c]imidazole-7-yl)-N-methyl-2-napthamide; N-ethyl-6-(7-hydroxy-6,7-dihydro-5*H*-pyrrolo[1,2-c]imidazole-7-yl)-2-napthamide; N-cyclopropyl-6-(7-hydroxy-6,7-dihydro-5*H*-pyrrolo[1,2-c]imidazole-7-yl)-2-napthamide; 6-(7-hydroxy-6,7-dihydro-5*H*-pyrrolo[1,2-c]imidazole-7-yl)-N-isopropyl-2-napthamide; N,N-diisopropyl-6-(7-hydroxy-6,7-dihydro-5*H*-pyrrolo[1,2-c]imidazole-7-yl)-2-napthamide; 6-[1-hydroxy-1-(1-methyl-1*H*-imidazol-5-yl)ethyl]-N-methylnaphthalene-2-carboxamide; 6-(6,7-dihydro-5*H*-pyrrolo[1,2-c]imidazole-7-yl)-N-methyl-2-napthamide; and 6-(7-hydroxy-6,7-dihydro-6,6-dimethyl-5*H*-pyrrolo[1,2-c]imidazole-7-yl)-N-isopropyl-2-napthamide. See Kaku et al., Bioorg. Med. Chem. (2011) 19, 6383-99.

Moreover, preferred examples of selective 17,20-lyase inhibitors include orteronel and VT-464. *See* Kaku et al., Bioorg. Med. Chem. (2011) 19, 6383-99; Eisner et al. J. Clin. Oncol. "VT-464: A novel , selective inhibitor of P450c17(CYP17)-17,20 lyase for castration-refractory prostate cancer (CRPC).

One of skill in the art can readily determine additional examples of selective 17,20-lyase inhibitors by screening inhibitors of CYP17 for both 17,20-lyase inhibition and hydroxylase inhibition, such as 17α-hydroxylase inhibition. In some embodiments, a compound is a selective inhibitor if there is a 5-fold difference between lyase and hydroxylase inhibition. In other embodiments, a selective inhibitor will have at least a 10, 20, 30, 50, or 100-fold difference in inhibition. Methods to determine selective inhibition are known in the art.

In some embodiments, a hormonal therapy agent is selected from the group consisting of enzulatomide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); VT-464; orteronel; aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl}-3-oxo-4-azaandrost-1-ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18B-glycerrhetinic acid; altraric acid; N-butylbenzene-sulfonamide; and 3,3'-diindolylmethane. In other embodiments, the hormonal therapy agent is selected from the group consisting of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); and vinclozolin ((RS)-3-(3,5-dichlorophenyl)-5-methyl- 5-vinyloxazolidine-2,4-dione). In other embodiments, the hormonal therapy agent is selected from the group consisting of galeterone (17-(1H-benzimidazol-1-yl)androsta-5,16-dien-3B-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); VT-464; orteronel; aminoglutethimide; and prochloraz (N-propyl-N-[2-(2,4,6-trichlorophenoxy)ethyl]imidazole-1-carboxamide). In other embodiments, the hormonal therapy agent is selected from the group consisting of dutasteride (5a, 17β)-N-{2,5 bis(trifluoromethyl) phenyl}-3-oxo-4-azaandrost-1-ene-17-carboxamide); izonteride ((4aR,1 0bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); and epristeride (17-(tert-butylcarbamoyl)androsta-3,5-diene-3-carboxylic acid). In other embodiments, the hormonal therapy agent is selected from the group consisting of genisterin; gossypol; equol; 18B-glycerrhetinic acid; altraric acid; N-butylbenzene-sulfonamide; and 3,3'-diindolylmethane. In other embodiments, the hormonal therapy agent is selected from deslorelin; nafarelin; cetrorelix; and ganirelix

In some embodiments, the hormonal therapy agent is a luteinizing hormone-releasing hormone (LHRH) antagonist or agonist. In some embodiments, the hormonal therapy agent is a gonadotropin-releasing hormone agonist. In some embodiments, the hormonal therapy agent is a gonadotropin-releasing hormone agonist selected from deslorelin or nafarelin. In some embodiments, the hormonal therapy agent is a gonadotropin-releasing hormone antagonist. In some embodiments, the hormonal therapy agent is a gonadotropin-releasing hormone antagonist selected from cetrorelix or ganirelix.

In some embodiments, one or more of the hormone therapy agents described above are administered to the patient before administering a compound of Formula (I). In other embodiments, one or more of the hormone therapy agents described above are administered to the patient after administering a compound of Formula (I). In other embodiments, one or more of the hormone therapy agents described above are concurrently (within a few hours) administered to the patient with a compound of Formula (I).

In some embodiments, the androgen that is decreased in the subject is testosterone, dihydrotestosterone (DHT), androsterone, androstenediol, androstenedione, dehydroepiandrosterone (DHEA), and dehydroepiandrosterone sulfate (DHEA-S).

Normal serum testosterone ranges between 1000-300 ng/dL. In some embodiments, a subject is provided a therapy, as described herein, whereby a reduction in the treated subject's serum testosterone level to at least about ≤ 80, ≤ 70, ≤ 60, ≤ 50, ≤ 40, ≤ 30, ≤ 20, or ≤ 10 ng/dL is obtained. In some embodiments, a subject is provided a therapy that reduces the subject's serum testosterone level to at least about ≤ 50 ng/dL. In some embodiments, a subject is treated with a therapy that results in a reduction in the subject's serum testosterone level to at least about ≤ 20 ng/dL. In some embodiments, a subject is treated with a therapy, as described herein, that reduces the subject's serum testosterone level to at least about or any number in between the range of 120-70, 100-60, 80-40, 70-30, 50-20, 40-10, 30-10, or 20-10 ng/dL. In some embodiments, a subject is treated with a therapy that produces a reduction in the subject's serum testosterone level to about ≤ 95%, ≤ 90%, ≤ 80%, ≤ 70%, ≤ 60%, or ≤ 50% that of a healthy male. In some embodiments, a subject is treated with a therapy that results in a reduction in the subject's serum testosterone level to the range of at least about or any number in between the range of about 5-20%, 10-30%, 20-40%, 30-50%, 40-60%, or 50-70% that of a healthy male.

In some embodiments, a subject suffering from prostate cancer is classified, selected, or identified as a subject in need of a therapy for prostate cancer. Optionally, the inhibition in prostate cancer cell growth or an inhibition in prostate cancer advancement is evaluated. Optionally, the delaying prostate cancer cell growth or delaying prostate cancer advancement is evaluated. A subject can be identified as one in need of a therapy for prostate cancer using conventional clinical pathology including, biopsy, CT scan, MRI, digital examination, Gleason score, or PSA level. Patients today also get PET scans, which are very important since they evaluate the activity of the tumor cells (glucose metabolism). Similarly, the inhibition or delay of cancer cell growth in said subject after receiving the treatment can be evaluated using conventional clinical pathology including, biopsy, CT scan, MRI, digital examination, Gleason score, or PSA level.

As mentioned above, prostate cancer can be treated by hormone therapy agents, however, hormone therapy agents alone can result in the development of castration-resistant prostate cancer (CRPC). For example, hormonal therapy can initially deliver a response in a subject suffering from prostate cancer, however, the return of hormone-refractory tumors invariably prevents long-term patient survival. More effective strategies are needed to extend life expectancy and improve the quality of life for patients with advanced prostate cancer. Accordingly, some aspects of the present invention concern methods for ameliorating or inhibiting or reducing or delaying the onset of castration-resistant prostate cancer (CRPC) or treatments (e.g., compositions used for the purpose of ameliorating or inhibiting or reducing or delaying the onset of CRPC), whereby one or more of the compounds of Formula (I) (e.g., a compound from Table 1) are provided before, during and/or after providing one or more of the hormone therapy agents described above Optionally, the inhibition in prostate cancer cell growth, an inhibition in prostate cancer advancement, or delaying the onset of CRPC is evaluated. Optionally, a patient with prostate cancer is classified as a subject in need of an agent that ameliorates, reduces, delays, or inhibits the onset of CRPC prior to receiving one or more of the combination therapies described herein. A subject can be identified as one in need of a therapy for prostate cancer using conventional clinical pathology including, biopsy, CT scan, MRI, digital examination, Gleason score, or PSA level.

Accordingly, aspects of the disclosure concern the use of diagnostic and/or clinical tools in conjunction with the administration of a hormone therapy agent and plumbagin. That is in some embodiments, a patient with prostate cancer but not CRPC is identified using clinical or diagnostic methods and said identified patient is provided a testosterone synthesis inhibitor that does not bind to the androgen receptor, such as orteronel or VT-464, preferably before, during and/or after administration of plumbagin. In other embodiments, a patient with CRPC is identified using clinical or diagnostic methods and said identified patient is provided a testosterone synthesis inhibitor that does not bind to the androgen receptor, such as orteronel or VT-464 before, during, and/or after administration of plumbagin. The clinical and/or diagnostic methods, which are companion to the therapies described herein can be practiced before, during or after administration of the hormone therapy agent and/or the plumbagin.

For example, the inhibition or delay of cancer cell growth or the presence of prostate cancer or CRPC in said subject before, during, or after receiving the hormone therapy agent and/or plumbagin can be evaluated using conventional clinical pathology including, biopsy, CT scan, MRI, digital examination, Gleason score, or PSA level. The section below describes these therapies in greater detail.

### IV. Combination Therapies

In some embodiments, the compounds disclosed herein, such as a compound of Formula (I) (e.g., a compound of Table 1), or a pharmaceutically acceptable salt thereof, or a pharmaceutical composition that includes a compound described herein, can be used in combination with one or more hormone therapy agents, preferably a testosterone synthesis inhibitor that does not bind to the androgen receptor, such as orteronel or VT-464. Some embodiments disclosed herein relate to a method of ameliorating or treating a neoplastic disease that can include administering to a subject suffering from a neoplastic disease a therapeutically effective amount of one or more compounds described herein (e.g., a compound of Formula (I), or a pharmaceutically acceptable salt thereof), in combination with one or more hormone therapy agents, such as a testosterone synthesis inhibitor that does not bind to the androgen receptor, e.g., orteronel or VT-464.

In some embodiments, the neoplastic disease can be cancer. In some embodiments, the neoplastic disease can be a tumor such as a solid tumor. In an embodiment, the neoplastic disease can be prostate cancer and in some embodiments the prostate cancer can be CRPC. In some embodiments, the prostate cancer is androgen dependent. Therefore, in some embodiments, a compound of Formula (I), or a pharmaceutically acceptable salt thereof, or a pharmaceutical composition that includes a compound of Formula (I), or a pharmaceutically acceptable salt thereof, is used in combination with one or more hormone therapy agents (e.g., a testosterone synthesis inhibitor that does not bind to the androgen receptor, such as orteronel or VT-464) for the use in treating, inhibiting, delaying, or ameliorating progression of prostate cancer, growth of prostate cancer cells, or for inhibiting or preventing the onset of androgen-dependent prostate cancer, or for decreasing the size of a prostate tumor, or for inhibiting the onset of metastic prostate cancer. In some embodiments, a compound of Formula (I), or a pharmaceutically acceptable salt thereof, or a pharmaceutical composition that includes a compound of Formula (I), or a pharmaceutically acceptable salt thereof, is used in combination with one or more hormone therapy agents (e.g., a testosterone synthesis inhibitor that does not bind to the androgen receptor, such as orteronel or VT-464) for the use in increasing the survival rate of a patient suffering from prostate cancer. In some embodiments, a compound of Formula (I), or a pharmaceutically acceptable salt thereof, or a pharmaceutical composition that includes a compound of Formula (I), or a pharmaceutically acceptable salt thereof, is used in combination with one or more hormone therapy agents (e.g., a testosterone synthesis inhibitor that does not bind to the androgen receptor, such as orteronel or VT-464) for the use in increasing the survival rate of a patient suffering from CRPC. In some embodiments, a compound of Formula (I), or a pharmaceutically acceptable salt thereof, or a pharmaceutical composition that includes a compound of Formula (I), or a pharmaceutically acceptable salt thereof, is used in combination with one or more hormone therapy agents (e.g., a testosterone synthesis inhibitor that does not bind to the androgen receptor, such as orteronel or VT-464) for the use in curing a patient suffering from prostate cancer.

Intermittent hormonal therapy (IHT) is an alternative to continuous hormonal therapy, which may delay progression of hormone-refractory disease (i.e., CRPC). For example, intermittent therapy can be used for a period of 6 months on, followed by a period of 6 months off. In some embodiments, one or more hormonal therapy agents is provided for one month on, followed by one month off. In some embodiments, one or more hormonal therapy agents is provided for three months on, followed by three months off. Accordingly, one or more of the compounds of Formula (I), e.g., a compound of Table 1, can be provided before, during and/or after administering one or more hormonal therapy agents (e.g., a testosterone synthesis inhibitor that does not bind to the androgen receptor, such as orteronel or VT-464), as described above, so as to reduce or inhibit or delay the onset of CRPC.

A non-limiting list of example combination of compounds of Formula (I), or a pharmaceutically acceptable salt thereof, or a pharmaceutical composition that includes a compound described herein, with one or more hormonal therapy agents are provided in Tables 1 and 2. Table 1 provides a shorthand name for each compound of Formula (I) and a shorthand name for each hormonal therapy agent. Each numbered X compound in Table 2 has a corresponding compound structure provided in Table 1. Likewise, each numbered Y therapy in Table 2 has a corresponding therapy provided in Table 1. Therefore, each "X:Y" entry in Table 2 provides an example of a combination of a compound of Formula (I) and a hormonal therapy agent that can be used to treat a subject suffering from prostate cancer. For example, the combination designated as "F02:AT04" in Table 2 provides a combination of 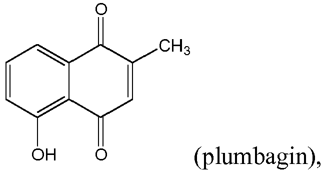 and ARN-509 that can be used to treat a subject suffering from prostate cancer. Each of the combinations provided in Table 2 can be used with one, two, three or more additional agents described herein.

**Table 1**

| **Compound of Formula (I)** | | **Additional Therapy** | |
|---|---|---|---|
| 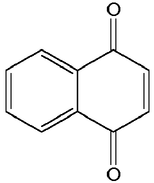 | (F01) | orteronel | (AT01) |
| 1,4-naphthoquinone | | | |
| 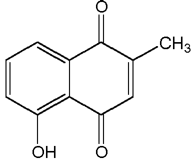 | (F02) | VT-464 | (AT02) |
| plumbagin | | | |
| 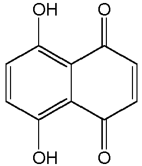 | (F03) | enzalutamide | (AT03) |
| naphthazarin | | | |
| 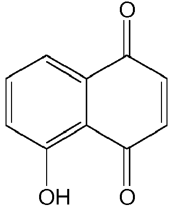 | (F04) | ARN-509 | (AT04) |
| juglone | | | |
| 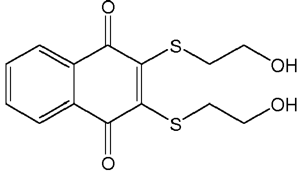 | (F05) | vinclozolin | (AT05) |
| NSC 95397 | | | |
| 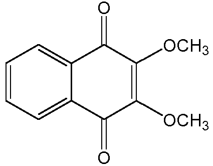 | (F06) | galeterone | (AT06) |
| DMNQ | | | |
| 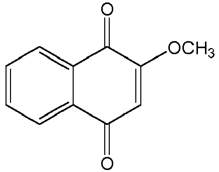 | (F07) | ketoconazole | (AT07) |
| 2-methoxy-1,4,naphthoquinone | | | |
| 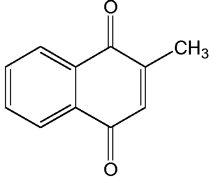 | (F08) | L-39 | (AT08) |
| menadione | | | |
| 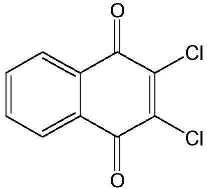 | (F09) | aminoglutethi mide | (AT09) |
| dichlon | | | |
| 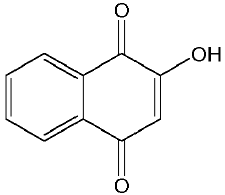 | (F10) | prochloraz | (AT10) |
| lawsone | | | |
| 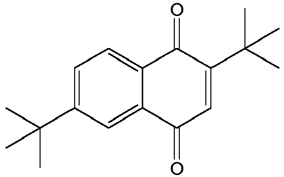 | (F11) | dutasteride | (AT 11) |
| 2,6-di-tert-butyl-1,4-naphtoquinone | | | |
| 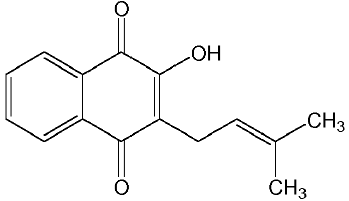 | (F12) | izonteride | (AT12) |
| lapachol | | | |
| 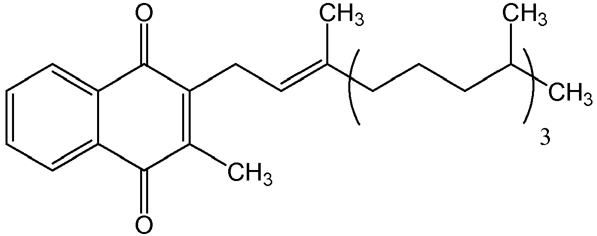 | (F13) | turosteride | (AT13) |
| phylloquinone | | | |
| 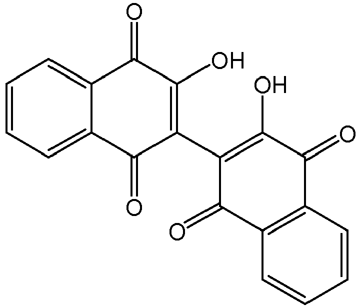 | (F14) | epristeride | (AT14) |
| lawson dimer | | | |
| -- | | genisterin | (AT15) |
| - | | gossypol | (AT16) |
| -- | | equol | (AT17) |
| -- | | 18β-glycerrhetinic acid | (AT18) |
| -- | | altraric acid | (AT19) |
| -- | | N-butylbenzene-sulfonamide | (AT20) |
| -- | | 3,3'-diindolylmeth ane | (AT21) |
| -- | | deslorelin | (AT22) |
| -- | | nafarelin | (AT23) |
| -- | | cetrorelix | (AT24) |
| -- | | ganirelix | (AT25) |

**Table 2**

| X : Y | X : Y | X : Y | X : Y | X : Y | X : Y | X : Y |
|---|---|---|---|---|---|---|
| F01 : AT02 | F02 : AT02 | F03 : AT02 | F04 : AT02 | F05 : AT02 | F06 : AT02 | F07 : AT02 |
| F01 : AT03 | F02 : AT03 | F03 : AT03 | F04 : AT03 | F05 : AT03 | F06 : AT03 | F07 : AT03 |
| F01 : AT04 | F02 : AT04 | F03 : AT04 | F04 : AT04 | F05 : AT04 | F06 : AT04 | F07 : AT04 |
| F01 : AT05 | F02 : AT05 | F03 : AT05 | F04 : AT05 | F05 : AT05 | F06 : AT05 | F07 : AT05 |
| F01 : AT06 | F02 : AT06 | F03 : AT06 | F04 : AT06 | F05 : AT06 | F06 : AT06 | F07 : AT06 |
| F01 : AT07 | F02 : AT07 | F03 : AT07 | F04 : AT07 | F05 : AT07 | F06 : AT07 | F07 : AT07 |
| F01 : AT08 | F02 : AT08 | F03 : AT08 | F04 : AT08 | F05 : AT08 | F06 : AT08 | F07 : AT08 |
| F01 : AT09 | F02 : AT09 | F03 : AT09 | F04 : AT09 | F05 : AT09 | F06 : AT09 | F07 : AT09 |
| F01 : AT10 | F02 : AT10 | F03 : AT10 | F04 : AT10 | F05 : AT10 | F06 : AT10 | F07 : AT10 |
| F01 : AT11 | F02 : AT11 | F03 : AT11 | F04 : AT11 | F05 : AT11 | F06 : AT11 | F07 : AT11 |
| F01 : AT12 | F02 : AT12 | F03 : AT12 | F04 : AT12 | F05 : AT12 | F06 : AT12 | F07 : AT12 |
| F01 : AT13 | F02 : AT13 | F03 : AT13 | F04 : AT13 | F05 : AT13 | F06 : AT13 | F07 : AT13 |
| F01 : AT14 | F02 : AT14 | F03 : AT14 | F04 : AT14 | F05 : AT14 | F06 : AT14 | F07 : AT14 |
| F01 : AT15 | F02 : AT15 | F03 : AT15 | F04 : AT15 | F05 : AT15 | F06 : AT15 | F07 : AT15 |
| F01 : AT16 | F02 : AT16 | F03 : AT16 | F04 : AT16 | F05 : AT16 | F06 : AT16 | F07 : AT16 |
| F01 : AT17 | F02 : AT17 | F03 : AT17 | F04 : AT17 | F05 : AT17 | F06 : AT17 | F07 : AT17 |
| F01 : AT18 | F02 : AT18 | F03 : AT18 | F04 : AT18 | F05 : AT18 | F06 : AT18 | F07 : AT18 |
| F01 : AT19 | F02 : AT19 | F03 : AT19 | F04 : AT19 | F05 : AT19 | F06 : AT19 | F07 : AT19 |
| F01 : AT20 | F02 : AT20 | F03 : AT20 | F04 : AT20 | F05 : AT20 | F06 : AT20 | F07 : AT20 |
| F01 : AT21 | F02 : AT21 | F03 : AT21 | F04 : AT21 | F05 : AT21 | F06 : AT21 | F07 : AT21 |
| F01 : AT22 | F02 : AT22 | F03 : AT22 | F04 : AT22 | F05 : AT22 | F06 : AT22 | F07 : AT22 |
| F01 : AT23 | F02 : AT23 | F03 : AT23 | F04 : AT23 | F05 : AT23 | F06 : AT23 | F07 : AT23 |
| F01 : AT24 | F02 : AT24 | F03 : AT24 | F04 : AT24 | F05 : AT24 | F06 : AT24 | F07 : AT24 |
| F01 : AT25 | F02 : AT25 | F03 : AT25 | F04 : AT25 | F05 : AT25 | F06 : AT25 | F07 : AT25 |
| F08 : AT02 | F09 : AT02 | F10 : AT02 | F11 : AT02 | F12 : AT02 | F13 : AT02 | F14 : AT02 |
| F08 : AT03 | F09 : AT03 | F10 : AT03 | F11 : AT03 | F12 : AT03 | F13 : AT03 | F14 : AT03 |
| F08 : AT04 | F09 : AT04 | F10 : AT04 | F11 : AT04 | F12 : AT04 | F13 : AT04 | F14 : AT04 |
| F08 : AT05 | F09 : AT05 | F10 : AT05 | F11 : AT05 | F12 : AT05 | F13 : AT05 | F14 : AT05 |
| F08 : AT06 | F09 : AT06 | F10 : AT06 | F11 : AT06 | F12 : AT06 | F13 : AT06 | F14 : AT06 |
| F08 : AT07 | F09 : AT07 | F10 : AT07 | F11 : AT07 | F12 : AT07 | F13 : AT07 | F14 : AT07 |
| F08 : AT08 | F09 : AT08 | F10 : AT08 | F11 : AT08 | F12 : AT08 | F13 : AT08 | F14 : AT08 |
| F08 : AT09 | F09 : AT09 | F10 : AT09 | F11 : AT09 | F12 : AT09 | F13 : AT09 | F14 : AT09 |
| F08 : AT10 | F09 : AT10 | F10 : AT10 | F11 : AT10 | F12 : AT10 | F13 : AT10 | F14 : AT10 |
| F08 : AT11 | F09 : AT11 | F10 : AT11 | F11 : AT11 | F12 : AT11 | F13 : AT11 | F14 : AT11 |
| F08 : AT12 | F09 : AT12 | F10 : AT12 | F11 : AT12 | F12 : AT12 | F13 : AT12 | F14 : AT12 |
| F08 : AT13 | F09 : AT13 | F10 : AT13 | F11 : AT13 | F12: AT13 | F13 : AT13 | F14 : AT13 |
| F08 : AT14 | F09 : AT14 | F10 : AT14 | F11: AT14 | F12 : AT14 | F13 : AT14 | F14 : AT14 |
| F08 : AT15 | F09 : AT15 | F10 : AT15 | F11 : AT15 | F12 : AT15 | F13 : AT15 | F14 : AT15 |
| F08 : AT16 | F09 : AT16 | F10 : AT16 | F11 : AT16 | F12: AT16 | F13 : AT16 | F14 : AT16 |
| F08 : AT17 | F09 : AT17 | F10 : AT17 | F11: AT17 | F12 : AT17 | F13 : AT17 | F14 : AT17 |
| F08 : AT18 | F09 : AT18 | F10 : AT18 | F11 : AT18 | F12: AT18 | F13 : AT18 | F14 : AT18 |
| F08 : AT19 | F09 : AT19 | F10 : AT19 | F11 : AT19 | F12 : AT19 | F13 : AT19 | F14 : AT19 |
| F08 : AT20 | F09 : AT20 | F10 : AT20 | F11 : AT20 | F12 : AT20 | F13 : AT20 | F14 : AT20 |
| F08 : AT21 | F09 : AT21 | F10 : AT21 | F11 : AT21 | F12 : AT21 | F13 : AT21 | F14 : AT21 |
| F08 : AT22 | F09 : AT22 | F10 : AT22 | F11 : AT22 | F12 : AT22 | F13 : AT22 | F14 : AT22 |
| F08 : AT23 | F09 : AT23 | F10 : AT23 | F11 : AT23 | F12 : AT23 | F13 : AT23 | F14 : AT23 |
| F08 : AT24 | F09 : AT24 | F10 : AT24 | F11 : AT24 | F12 : AT24 | F13 : AT24 | F14 : AT24 |
| F08 : AT25 | F09 : AT25 | F10 : AT25 | F11 : AT25 | F12 : AT25 | F13 : AT25 | F14 : AT25 |

The order of administration of a compound of Formula (I), or a pharmaceutically acceptable salt thereof, with one or more additional hormone therapy agents (e.g., a testosterone synthesis inhibitor that does not bind to the androgen receptor, such as orteronel or VT-464) can vary. In some embodiments, a compound of Formula (I), or a pharmaceutically acceptable salt thereof, can be administered prior to all additional hormone therapy agents (e.g., a testosterone synthesis inhibitor that does not bind to the androgen receptor, such as orteronel or VT-464). In other embodiments, a compound of Formula (I), or a pharmaceutically acceptable salt thereof, can be administered prior to at least one additional hormone therapy agent (e.g., a testosterone synthesis inhibitor that does not bind to the androgen receptor, such as orteronel or VT-464). In still other embodiments, a compound of Formula (I), or a pharmaceutically acceptable salt thereof, can be administered concomitantly with one or more additional hormone therapy agents (e.g., a testosterone synthesis inhibitor that does not bind to the androgen receptor, such as orteronel or VT-464). In yet still other embodiments, a compound of Formula (I), or a pharmaceutically acceptable salt thereof, can be administered subsequent to the administration of at least one additional hormone therapy agent (e.g., a testosterone synthesis inhibitor that does not bind to the androgen receptor, such as orteronel or VT-464). In some embodiments, a compound of Formula (I), or a pharmaceutically acceptable salt thereof, can be administered subsequent to the administration of all additional hormone therapy agents (e.g., a testosterone synthesis inhibitor that does not bind to the androgen receptor, such as orteronel or VT-464).

In some embodiments, the compounds disclosed herein, such as a compound of Formula (I) (e.g., a compound of Table 1), or a pharmaceutically acceptable salt thereof, or a pharmaceutical composition that includes a compound described herein, can be used in combination with one or more hormone therapy agents (preferably a testosterone synthesis inhibitor that does not bind to the androgen receptor, such as orteronel or VT-464) and in further combination with one or more statins. Statins are inhibitors of HMG-CoA reductase that can be administered to a subject to reduce testosterone/dihydrotestosterone levels. In some embodiments, a compound of Formula (I), or a pharmaceutically acceptable salt thereof, or a pharmaceutical composition that includes a compound described herein, can be used in combination with one or more statins. In some embodiments, the one or more statins can be selected from among simvastatin (Zocor), atrovastatin (Lipitor), fluvastatin (Lescol), lovastatin (Mevacor, Altocor), pitavastatin (Livalo), pravastatin (Pravachol), and rosuvastatin (Crestor).

In some embodiments, the compounds disclosed herein, such as a compound of Formula (I) (e.g., a compound of Table 1), or a pharmaceutically acceptable salt thereof, or a pharmaceutical composition that includes a compound described herein, can be used in combination with one or more hormone therapy agents (preferably a testosterone synthesis inhibitor that does not bind to the androgen receptor, such as orteronel or VT-464) and in further combination with Ezatimibe (Zetia, Ezetrol, (3R,4S)-1-(4-fluorophenyl)-3-[(3S)-3-(4-fluorophenyl)-3-hydroxypropyl]-4-(4-hydroxyphenyl)-azetidin-2-one). In some embodiments, a compound of Formula (I), or a pharmaceutically acceptable salt thereof, or a pharmaceutical composition that includes a compound described herein, can be used in combination with Ezatimibe. Ezatimibe can decrease a subject's cholesterol absorption. In some embodiments, a compound of Formula (I) can be used in combination with one or more hormone therapy agents described herein, in further combination with a statin described herein, and in further combination with Ezatimibe.

### V. Methods of Compound Identification

Some embodiments are directed to methods that facilitate drug discovery. Some embodiments are directed to methods of identifying compounds that induce cell cycle entry. Some embodiments, are directed to method of identifying compounds that induce mitosis in a cell line. The cell or cell line may be a cancer cell line, such as prostate cancer, or androgen dependent prostate cancer, or CRPC. Some embodiments are directed to identifying compounds that induce or act as growth factors for a cell line. The cell line may be a cancer cell line, such as prostate cancer, or androgen dependent prostate cancer, or CRPC.

Some embodiments are directed to a method of identifying a compound that, preferably concomitantly, induces growth of androgen-dependent prostate cancer cells in the absence of dihydrotestosterone and induces apoptosis of said androgen-dependent prostate cancer cells. These methods can be practiced by reducing the amount of dihydrotestosterone in contact with a population of cells comprising androgen-dependent prostate cancer cells, preferably using a compound that selectively inhibits testosterone synthesis without interacting with the androgen receptor or removing dihydrotestosterone from the media of prostate cells in culture; administering one or more candidate compounds to the androgen-starved androgen-dependent prostate cancer cells; measuring cell cycle entry or specific phases of the cell cycle (i.e. mitosis) in the androgen-dependent prostate cancer cells; and optionally, measuring the amount of cell death or apoptosis of said androgen-dependent prostate cancer cells, wherein an increase in cell cycle entry or an increase in specific phases of the cell cycle (i.e. cellular mitosis) relative to control cells and optionally, an increase in cell death or apoptosis of said androgen-dependent prostate cancer cells indicates that the compound acts as an inducer of prostate cancer cell cycle entry and, optionally, an inducer of prostate cancer cell death or apoptosis. In some embodiments, the androgen-dependent prostate cancer cells are in culture. In some embodiments, the androgen-dependent prostate cancer cells are in a pseudo-orthotopic chamber mouse model. Some embodiments further comprise identifying compounds that are both a growth factor and cytotoxic to a cell line, such as a prostate cancer cell line or an androgen-dependent prostate cancer cells. In some embodiments, the identification of cytotoxicity involves monitoring the apoptosis of cells having increased cell cycle entry or increased incidence of specific phases of the cell cycle, such as mitosis (e.g., caspase activity). In some embodiments, the identified compounds are further measured for their ability to affect microtubule polymerization. In some embodiments, cell cycle entry is measured by flow cytometry. In some embodiments, a specific phase of the cell cycle, such as mitosis, is measured by flow cytometry. In some embodiments, an inhibitor of cytochrome P450-17 (CYP17), a 5α-reductase inhibitor, or both are administered to reduce the dihydrotestosterone levels. In some embodiments, a selective inhibitor of the 17,20-lyase activity of cytochrome P450-17 (CYP17), a 5α-reductase inhibitor, or both are administered to reduce the dihydrotestosterone levels. In some embodiments, manipulation of the cells' serum is used to affect dihydrotestosterone levels. Manipulation of the cells' serum includes removing dihydrotestorone-containing serum from the cells.

Some embodiments are directed to a method of identifying a compound that induces androgen-dependent prostate cancer cells to enter cell cycle in the absence of dihydrotestosterone, comprising: reducing the amount of dihydrotestosterone that is in contact with a population of androgen-dependent prostate cancer cells; contacting said population of androgen-dependent prostate cancer cells with a candidate compound; and determining or measuring whether said population of androgen-dependent prostate cancer cells enters cell cycle, a specific phase of the cell cycle, or mitosis after contact with said candidate compound, wherein said compound is identified as one that induces androgen-dependent prostate cancer cells to enter cell cycle in the absence of dihydrotestosterone when said population of androgen-dependent prostate cancer cells enters cell cycle, a specific phase of the cell cycle, or mitosis after contact with said candidate compound. In some embodiments, the androgen-dependent prostate cancer cells are in cell culture or a pseudo-orthotopic chamber mouse model. In some embodiments, the method further comprises determining whether said compound that induces androgen-dependent prostate cancer cells to enter cell cycle in the absence of dihydrotestosterone is cytotoxic to said androgen-dependent prostate cancer cells. In some embodiments, a cellular cytotoxicity assay is used to determine if said identified compound is cytotoxic to said androgen-dependent prostate cancer cells. In some embodiments, the cytotoxicity assay evaluates tubulin polymerization. In some embodiments, cell cycle entry, a specific phase of cell cycle, or mitosis is evaluated using flow cytometry. In some embodiments, an inhibitor of cytochrome P450-17 (CYP17), a 5α-reducase inhibitor, or both are administered to reduce the dihydrotestosterone levels. In some embodiments, a selective inhibitor of the 17,20-lyase activity of cytochrome P450-17 (CYP17), a 5α-reducase inhibitor, or both are administered to reduce the dihydrotestosterone levels. In some embodiments, a selective inhibitor of the 17,20-lyase activity of cytochrome P450-17 (CYP17) is administered to reduce the dihydrotestosterone levels. In some embodiments, a 5α-reducase inhibitor is administered to reduce the dihydrotestosterone levels. In some embodiments, a hormonal therapy agent selected from the group consisting of deslorelin; nafarelin; cetrorelix; and ganirelix is administered to reduce the dihydrotestosterone levels. In some embodiments, serum is removed from the cells to reduce dihydrotestosterone levels. In some embodiments, said candidate compound is a compound of Formula (I), or a pharmaceutically acceptable salt of Formula (I): 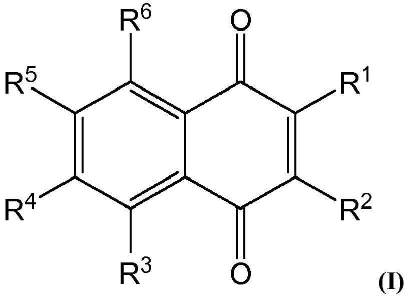 wherein: R¹ is selected from the group consisting of hydrogen, halogen, an optionally substituted C₁₋₁₈ alkyl, an optionally substituted C₂₋₁₈ alkenyl, -OR⁷ and -SR⁸; R² is selected from the group consisting of hydrogen, halogen, an optionally substituted C₁₋₆ alkyl, an optionally substituted C₂₋₆ alkenyl, -OR⁹ and -SR¹⁰; R³ is selected from the group consisting of hydrogen, an optionally substituted C₁₋₆ alkyl, and -OR¹¹; R⁴ is selected from the group consisting of hydrogen, an optionally substituted C₁₋₆ alkyl, and -OR¹²; R⁵ is selected from the group consisting of hydrogen, an optionally substituted C₁₋₆ alkyl, and - OR¹³; R⁶ is selected from the group consisting of hydrogen, an optionally substituted C₁₋₆ alkyl, and -OR¹⁴; and R⁷, R⁸, R⁹, R¹⁰, R¹¹, R¹², R¹³, and R¹⁴ are independently selected from hydrogen and an optionally substituted C₁₋₆ alkyl. In some embodiments, R¹, R², R³, R⁴, R⁵ and R⁶ are each hydrogen. In some embodiments, R¹ is methyl; R³ is -OH; and R², R⁴, R⁵ and R⁶ are each hydrogen. In some embodiments, R³ and R⁶ are each -OH; and R¹, R², R⁴ and R⁵ are each hydrogen. In some embodiments, R³ is -OH; and R¹, R², R⁴, R⁵ and R⁶ are each hydrogen. In some embodiments, R¹ and R² are each -SCH₂CH₂OH; and R³, R⁴, R⁵ and R⁶ are each hydrogen. In some embodiments, R¹ and R² are each -OCH₃; and R³, R⁴, R⁵ and R⁶ are each hydrogen. In some embodiments, R¹ is -OCH₃; and R², R³, R⁴, R⁵ and R⁶ are each hydrogen. In some embodiments, R¹ is methyl; and R², R³, R⁴, R⁵ and R⁶ are each hydrogen. In some embodiments, R¹ and R² are each chloro; and R³, R⁴, R⁵ and R⁶ are each hydrogen. In some embodiments, R¹ is -OH; and R², R³, R⁴, R⁵ and R⁶ are each hydrogen. In some embodiments, R¹ is phytenyl; R² is methyl; and R³, R⁴, R⁵ and R⁶ are each hydrogen. In some embodiments, R¹ and R⁴ are each t-butyl; and R², R³, R⁵ and R⁶ are each hydrogen. In some embodiments, R¹ is -OH; R² is -CH₂-CH=C(CH₃)₂; and R³, R⁴, R⁵ and R⁶ are each hydrogen. In some embodiments, the compound of Formula (I) is a selected from among the group consisting of: the compound where R¹, R², R³, R⁴, R⁵ and R⁶ are each hydrogen; the compound where R³ and R⁶ are each -OH; and R¹, R², R⁴ and R⁵ are each hydrogen; the compound where R³ is -OH; and R¹, R², R⁴, R⁵ and R⁶ are each hydrogen; the compound where R¹ and R² are each -SCH₂CH₂OH; and R³, R⁴, R⁵ and R⁶ are each hydrogen; the compound where R¹ and R² are each -OCH₃; and R³, R⁴, R⁵ and R⁶ are each hydrogen; the compound where R¹ is -OCH₃; and R², R³, R⁴, R⁵ and R⁶ are each hydrogen; the compound where R¹ is methyl; and R², R³, R⁴, R⁵ and R⁶ are each hydrogen; the compound where R¹ and R² are each chloro; and R³, R⁴, R⁵ and R⁶ are each hydrogen; the compound where R¹ is -OH; and R², R³, R⁴, R⁵ and R⁶ are each hydrogen; the compound where R¹ is phytenyl; R² is methyl; and R³, R⁴, R⁵ and R⁶ are each hydrogen; the compound where R¹ and R⁴ are each t-butyl; and R², R³, R⁵ and R⁶ are each hydrogen; and the compound where R¹ is -OH; R² is -CH₂-CH=C(CH₃)₂; and R³, R⁴, R⁵ and R⁶ are each hydrogen.

### VI. Determining and evaluating anti-cancer activity

### Animal models

Animal models are pivotal to further our understanding of the mechanisms of (progressive) growth of cancer. Currently used rodent tumor models, including transgenic tumor models, (using genetically modified mice susceptible to develop cancer), as well as implantation of human tumors under the skin in immunodeficient mice, do not sufficiently represent clinical cancer, especially with regard to metastasis and drug sensitivity. Preclinical tumor model systems employed to evaluate potential new treatment strategies should aim to represent the process and patterns of metastasis of their clinical counterparts as closely as possible.

A syngeneic pseudo-orthotopic *in vivo* model was developed to study the early steps of prostate cancer. Chambers are surgically placed into the dorsal skinfold of male mice. Briefly, male mice (25-30 g body weight) are anesthetized and placed on a heating pad. Two symmetrical titanium frames are implanted into the dorsal skinfold. A circular layer is excised from one of the skin layers. The underlying muscle and subcutaneous tissues are covered with a glass coverslip incorporated in one of the frames. After a recovery period of 2-3 days, stroma tissue and tumor cells are carefully placed in the chamber.

Tumor-derived cell lines can be grown directly in the chamber, corresponding to the traditional subcutaneous model. However, it was found that various minced tissues implanted in the chambers survive and revascularize, and that tumor-derived cell lines adapt to these various stroma after co-implantation, which points to this approach as an orthotopic model as well as a model for initial steps in metastasis.

For example, mouse prostate tissue can be grafted in the chamber. The graft develops its own vasculature and serve as orthotopic stroma for the tumor. A small number of prostate cancer cells (e.g., TRAMP-C2 cells derived from a TRAMP mouse) can be implanted on top of the prostate stroma. The tumor microenvironment can be important for the progression of different types of cancer, and orthotopic implantation of cancer cells can recapitulate human disease much more closely than subcutaneous implantation. Tumors can grow faster and develop better vasculature when the cancer cells are implanted into the relevant organ. Co-implanting mouse prostate cancer cells with prostate stroma can provide the tumor cells with an environment that better reflects the clinical disease compared to purely subcutaneous models. Re-vascularized stromal tissue and implanted tumors can remain viable for long periods of time using this method, for example, up to 90 days.

### Phosphate and tensin homolog (PTEN) deficient model

Mouse cells derived from the PTEN (phosphatase and tensin homolog deleted in chromosome 10) deficient model of prostate cancer can be used to study prostate cancer. The tumor suppressor PTEN is one of the most frequently mutated genes in human prostate cancer. Loss of PTEN can result in constitutively high PI3-kinase and Akt activities, which may lead to increased migration, invasiveness, cell proliferation and survival. Loss of PTEN can play a major role in the pathogenesis of human prostate cancer. Alteration of at least one PTEN allele is observed in approximately 60% of primary tumors. Loss of PTEN can be associated with higher Gleason scores and poor prognosis, cancer progression toward hormone-independence, resistance to chemotherapy or to radiotherapy, and bone metastasis. PTEN-deficient mice have an increased incidence of cancer, similarly to the human genetic predisposition to cancer known as Cowden syndrome, which is caused by germline mutation in the *PTEN* gene. In these respects, the PTEN-deficient model appears to mimic human development quite closely. Thus, heterozygous disruption of the PTEN gene can result in spontaneous development of tumors in several tissues and prostatic intraepithelial neoplasia (PIN) lesions in the prostate. Prostate-specific homozygous loss of PTEN can be sufficient to induce prostate tumors, which can progress into metastatic disease. Heterozygous loss of PTEN, on the other hand, can cause PIN with a late latency.

Germline homozygous deletion of PTEN may result in embryonic lethality due to PTEN ablation. This can be overcome through the conditional inactivation of the gene using the Cre-LoxP system. A transgenic mouse can be generated that displays expression of the Cre recombinase specifically in the epithelial cells of the prostate through the use of the prostate-specific probasin promoter (PB-Cre4 mice). By crossing these animals with mice that have floxed *PTEN* alleles, it can be possible to generate both heterozygous and homozygous mice in which PTEN is deleted specifically in the prostate epithelium. Progression of prostate cancer in this model is very similar to the progression of prostate cancer as observed in humans. For example, in this model epithelial hyperplasia was observed, followed by dysplasia, PIN, invasive adenocarcinoma, and finally metastases to the lymph nodes and to the lung. Similar to human cancer, the PTEN-null mice first regress following androgen ablation, and then become androgen-independent.

Epithelial cell lines can be derived from a prostate tumor dissected from a homozygous PTEN^{L/L}/PBCre+ mouse. At least two clonal cell lines (PTEN-P2 and PTEN-P8) are heterozygous PTEN^{L/+}. The remaining allele can be silenced by forced expression of the Cre recombinase in vitro (PTEN-CaP2 and PTEN-CaP8 cells). Loss of the second allele can increase anchorage-independent growth and confer tumorigenesis in vivo. Spontaneous androgen-independence can occur in vivo, even though the PTEN-CaP2 and PTEN-CaP8 cells express the androgen receptor.

The implementation of PTEN prostate cells in the animal models disclosed herein can be highly relevant to human prostate cancer, and can allow detailed observation of the growth and/or regression of prostate tumors in response to different treatment regimens. Implantation in syngeneic mice respects many aspects of normal tumor growth. For example, two pairs of mouse prostate cancer cells (PTEN-P2/8 and PTEN-CaP2/8) can facilitate examination of metastasis in a mouse model of prostate cancer that is relevant to human cancer.

### IntraVital Microscopy (IVM)

IntraVital Microscopy (IVM) can be used to visualize tumors in animals and analyze various aspects of cancer physiology such as tumor vascularization, cell migration and metastasis. An advantage of IVM includes the real-time analysis of dynamic processes with single-cell resolution. IntraVital microscopy offers the possibility to follow tumor growth in a non-invasive, non-destructive manner. The application of IVM can be limited to animal models that bear visually accessible tumors. Therefore, the dorsal skinfold chamber model described above can be compatible with IVM. Using IVM can permit a number of parameters to be measured in living animals and as a function of time, including tumor growth, angiogenesis, infiltration by immune cells, tumor cell migration, cell cycle entry, mitosis (cell-division) and apoptosis (programmed cell death), all in the context of the host and in real time.

### VII. Pharmaceutical Compositions

Some embodiments described herein relate to a pharmaceutical composition, that can include a therapeutically effective amount of a compound of Formula (I), (e.g., a compound in Table 1) or a pharmaceutically acceptable salt thereof, and a hormone therapy agent (e.g., a compound in Table 1) and a pharmaceutically acceptable carrier, diluent, excipient or combination thereof. In some embodiments, the pharmaceutical composition can include a single diastereomer of a compound of Formula (I), or a pharmaceutically acceptable salt thereof, (for example, a single diastereomer is present in the pharmaceutical composition at a concentration of greater than 99% compared to the total concentration of the other diastereomers). In other embodiments, the pharmaceutical composition can include a mixture of diastereomers of a compound of Formula (I), or a pharmaceutically acceptable salt thereof. For example, the pharmaceutical composition can include a concentration of one diastereomer of > about 50%, ≥ 60%, ≥ 70%, ≥ 80%, ≥ 90%, ≥ 95%, or ≥ 98%, as compared to the total concentration of the other diastereomers. In some embodiments, the pharmaceutical composition includes a racemic mixture of diastereomers of a compound of Formula (I), or a pharmaceutically acceptable salt thereof.

The pharmaceutical compositions described herein can be administered to a human patient *per se*, or in pharmaceutical compositions where they are mixed with other active ingredients, as in combination therapy, or carriers, diluents, excipients or combinations thereof. Proper formulation is dependent upon the route of administration chosen. Techniques for formulation and administration of the compounds described herein are known to those skilled in the art.

The pharmaceutical compositions disclosed herein may be manufactured in a manner that is itself known, *e.g*., by means of conventional mixing, dissolving, granulating, dragee-making, levigating, emulsifying, encapsulating, entrapping or tableting processes. Additionally, the active ingredients are contained in an amount effective to achieve its intended purpose. Many of the compounds used in the pharmaceutical combinations disclosed herein may be provided as salts with pharmaceutically compatible counterions.

Multiple techniques of administering a compound and/or agent exist in the art including, but not limited to, oral, rectal, topical, aerosol, injection and parenteral delivery, including intramuscular, subcutaneous, intravenous, intramedullary injections, intrathecal, direct intraventricular, intraperitoneal, intranasal and intraocular injections.

One may also administer the compound and/or agent in a local rather than systemic manner, for example, via injection of the compound directly into the infected area, often in a depot or sustained release formulation. Furthermore, one may administer the compound and/or agent in a targeted drug delivery system, for example, in a liposome coated with a tissue-specific antibody. The liposomes will be targeted to and taken up selectively by the organ.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions that can include a compound and/or agent described herein formulated in a compatible pharmaceutical carrier may also be prepared, placed in an appropriate container, and labeled for treatment of an indicated condition.

### VII. Dosing

As will be readily apparent to one skilled in the art, the useful *in vivo* dosage to be administered and the particular mode of administration will vary depending upon the age, weight, the severity of the affliction, and mammalian species treated, the particular compounds employed, and the specific use for which these compounds are employed. The determination of effective dosage levels, that is the dosage levels necessary to achieve the desired result, can be accomplished by one skilled in the art using routine methods, for example, human clinical trials and *in vitro* studies.

The dosage may range broadly, depending upon the desired effects and the therapeutic indication. Alternatively dosages may be based and calculated upon the surface area of the patient, as understood by those of skill in the art. Although the exact dosage will be determined on a drug-by-drug basis, in most cases, some generalizations regarding the dosage can be made. The daily dosage regimen for an adult human patient may be, for example, an oral dose of between 0.01 mg and 3000 mg of each active ingredient, preferably between 1 mg and 700 mg, e.g. 5 to 200 mg. The dosage may be a single one or a series of two or more given in the course of one or more days, as is needed by the subject. In some embodiments, an active ingredient will be administered for a period of continuous therapy, for example for a week or more, or for months or years. In some embodiments, an active ingredient can be administered one time per day.

Multiple doses can be administered to a subject. For example, an active ingredient can be administered once per month, twice per month, three times per month, every other week (qow), once per week (qw), twice per week (biw), three times per week (tiw), four times per week, five times per week, six times per week, every other day (qod), daily (qd), twice a day (qid), or three times a day (tid), over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

In some embodiments, a compound of Formula (I) or a pharmaceutically acceptable salt thereof, and a hormone therapy agent can be cyclically administered to a patient. Cycling therapy involves the administration of a first active ingredient for a period of time, followed by the administration of a second active ingredient for a period of time and repeating this sequential administration. Cycling therapy can reduce the development of resistance to one or more therapies, avoid or reduce the side effects of one or more therapies, and/or improve the efficacy of treatment. In some embodiments, a compound of Formula (I) or a pharmaceutically acceptable salt thereof, and a hormone therapy agent are administered in a cycle of less than about 3 weeks, about once every two weeks, about once every 10 days, or about once every week. The number of cycles can be from 1 to 12 cycles, or from 2 to 10 cycles, or from 3 to 8 cycles.

The daily dosage regimen for an adult human patient may be the same or different for two active ingredients provided in combination. For example, a compound of Formula (I) can be provided at a dose of between 0.01 mg and 3000 mg, while a hormone therapy agent can be provided at a dose of between 1 mg and 700 mg. The dosage or each active ingredient can be, independently, a single one or a series of two or more given in the course of one or more days, as is needed by the subject. In some embodiments, the active ingredients will be administered for a period of continuous therapy, for example for a week or more, or for months or years. In some embodiments, a compound of Formula (I), or a pharmaceutically acceptable salt thereof, can be administered one time per day. In some embodiments, the hormone therapy agent can be administered once a week.

In instances where human dosages for active ingredients have been established for at least some condition, those same dosages may be used, or dosages that are between about 0.1% and 500%, more preferably between about 25% and 250% of the established human dosage. Where no human dosage is established, as will be the case for newly-discovered pharmaceutical compositions, a suitable human dosage can be inferred from ED₅₀ or ID₅₀ values, or other appropriate values derived from *in vitro* or *in vivo* studies, as qualified by toxicity studies and efficacy studies in animals.

In cases of administration of a pharmaceutically acceptable salt, dosages may be calculated as the free base. As will be understood by those of skill in the art, in certain situations it may be necessary to administer the active ingredients disclosed herein in amounts that exceed, or even far exceed, the above-stated, preferred dosage range in order to effectively and aggressively treat particularly aggressive diseases or infections.

Dosage amount and interval may be adjusted individually to provide plasma levels of the active moiety which are sufficient to maintain the modulating effects, or minimal effective concentration (MEC). The MEC will vary for each active ingredient but can be estimated from in vitro data. Dosages necessary to achieve the MEC will depend on individual characteristics and route of administration. However, HPLC assays or bioassays can be used to determine plasma concentrations. Dosage intervals can also be determined using MEC value. Compositions should be administered using a regimen which maintains plasma levels above the MEC for 10-90% of the time, preferably between 30-90% and most preferably between 50-90%. In cases of local administration or selective uptake, the effective local concentration of the drug may not be related to plasma concentration.

It should be noted that the attending physician would know how to and when to terminate, interrupt, or adjust administration due to toxicity or organ dysfunctions. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

Active ingredients disclosed herein can be evaluated for efficacy and toxicity using known methods. For example, the toxicology of a particular active ingredient, or of a subset of the active ingredients, sharing certain chemical moieties, may be established by determining *in vitro* toxicity towards a cell line, such as a mammalian, and preferably human, cell line. The results of such studies are often predictive of toxicity in animals, such as mammals, or more specifically, humans. Alternatively, the toxicity of particular compounds in an animal model, such as mice, rats, rabbits, or monkeys, may be determined using known methods. The efficacy of a particular active ingredient may be established using several recognized methods, such as *in vitro* methods, animal models, or human clinical trials. When selecting a model to determine efficacy, the skilled artisan can be guided by the state of the art to choose an appropriate model, dose, route of administration and/or regime.

### EXAMPLES

Additional embodiments are disclosed in further detail in the following examples, which are not in any way intended to limit the scope of the claims.

### Example 1

Compounds of Formula (I) can be prepared by methods known in the art. Additionally, many compounds of Formula (I) are naturally occurring organic compounds that can be isolated from plants. Furthermore, many compounds of Formula (I) are commercially available.

Plumbagin is soluble in alcohol, acetone, chloroform, benzene, and acetic acid. Plumbagin has been used in preparation with Ethanol (*in vitro*) and in preparation with DMSO (in vitro) or DMSO with PEG 30% (*in vivo*).

### Example 2

**Cell culture:** PTEN-P2/GFP are cells that stably express histone H2B-GFP fusion protein. Kanda et al. (Kanda T, Sullivan KF, Wahl GM. Histone-GFP fusion protein enables sensitive analysis of chromosome dynamics in living mammalian cells. Curr Biol 1998 Mar 26;8(7):377-85) developed a highly sensitive method for observing chromosome dynamics in living cells. They fused the human Histone H2B gene to the gene encoding the GFP, which was transfected into human HeLa cells to generate a stable line constitutively expressing H2B-GFP. The H2B-GFP fusion protein was incorporated into chromatin without affecting cell cycle progression. We have generated cDNA encoding a Histone H2B-GFP fusion protein under the 5'LTR in the LXRN retroviral cassette, and have introduced it into a number of humans, as well as, murine cancer cell lines by retroviral transduction.

Cells are grown in DMEM medium containing 10% FBS, 2mM L-glutamine, 100 U/ml penicillin/100 µg/ml streptomycin, insulin-selenium-transferrin (5 µg/ml insulin), and DHT 10⁻⁸M final. Androgen withdrawal is achieved by keeping the cells in phenol red-free DMEM medium containing 10% charcoal-treated FBS and the same supplements as in the normal medium except for DHT. Cells are maintained in a humidified incubator at 37°C and 5% CO₂. G418 (100 µg/ml) is added to maintain stable expression of H2B-GFP.

**Cell counting:** Cells in 12-well plates are washed once with PBS, detached using Trypsin, and transferred to a suspension vial in a final volume of 10ml PBS. Cells are counted using a COULTER™ Multisizer II instrument (Beckman Coulter Inc., Hialeah, FL) gated for the appropriate cell size and corrected for particulate debris.

**Animal model and surgical techniques:** Animal experiments have been approved as appropriate. All surgical procedures are performed in a sterile laminar flow hood. Dorsal skinfold chambers and surgical instruments are autoclaved before use. Saline used to keep tissue moist during surgical preparation is mixed with gentamicin (50 µl/ml).

Male Nude mice (25-35 g body weight) are anesthetized (7.3 mg ketamine hydrochloride and 2.3 mg xylazine /100 g body weight, i.p.) and placed on a heating pad. Two symmetrical titanium frames are implanted into a dorsal skinfold, so as to sandwich the extended double layer of skin. A 15 mm full thickness circular layer is excised. The underlying muscle (M. cutaneous max.) and subcutaneous tissues are covered with a glass coverslip incorporated in one of the frames. After a recovery period of 2-3 days, prostate tissue and cancer cell spheroids are carefully placed in the chamber. Small circular Band Aids are applied on the backside of the chamber after surgery to prevent scratching. Before surgery, Buprenorphine (0.1 mg/kg) will be given IP. After surgery Meloxicam will be given in the drinking water for 4 days Meloxicam (5.0 mg/ml), is added at 35 µl per 100ml of water to be medicated.

**Preparation of stroma:** A male donor mouse is euthanized and the anterior prostate tissue is excised, put in a Petri dish with antibiotics (gentamicin 50 µl/ml), and minced with fine scissors into small pieces (< 1 mm²) for implantation.

**Preparation of tumor spheroids:** Liquid overlay plates are generated using 1% Agarose melted in DMEM that is added to round-bottom 96-well plates (50ul/well). Cancer cells grown as pre-confluent monolayers are trypsinized, diluted to a final volume of 250,000 tumor cells/ml. Viability is determined using Trypan blue. The cells are plated at 100ul/well into the agarose-coated plates. After 48 hrs the cells form spheroids, which are picked and washed in serum-free medium before implantation into the mouse chambers. Viability is determined using Trypan blue. The size of the implanted spheroid can be determined precisely to minimize variations between animals.

**Surgical Castration:** Mice are anesthetized with 7.3 mg ketamine hydrochloride and 2.3 mg xylazine /100 g body weight, i.p. A lateral incision across the scrotum is made and the testes are individually ligated and excised. The wound was cauterized. The incision was then sutured and sealed with Nexaband® acrylic.

**Intravital microscopy:** Fluorescence microscopy is performed using a Mikron Instrument Microscope equipped with epi-illuminator and video-triggered stroboscopic illumination from a xenon arc (MV-7600, EG&G). A silicon intensified target camera (SIT68, Dage-MTI) is attached to the microscope. A Hamamatsu image processor (Argus 20) with firmware version 2.50 (Hamamatsu Photonic System) is used for image enhancement and for the capture of images to a computer. A Zeiss Plan Neoflour 1.25X/0.035 objective is used to obtain an over-view of the chamber and to determine tumor size. A Zeiss A-Plan 10X/0.25 objective is used to capture images for calculation of vascular parameters. A Zeiss Achroplan 20X/0.5 W objective is used to capture images for calculation of mitotic and apoptotic indices. Our system permits evaluation of the following parameters.

Tumor area (A_{T}) is defined as number of pixels with photo density above 75 (256 gray levels), i.e., **A_{T}**= ∑Aₖ, for 75< k < 255.

Number of Tumor cells: When tumors are heterogeneous, changes in **A_{T}** do not directly reflect tumor growth. An estimate of the number of tumor cells (**N_{TC}**) can be obtained by fitting to a quadratic function of an intensity index, e.g. **N**_{TC}= -3.296 x10⁻¹² + 190.6 x I_{T} + 7.7310⁻² x (I_{T})², where the index of intensity is given by **I_{T}** = ∑Aₖ∗k, for 75< k < 255.

Mitotic and Apoptotic Indices: At each time point, two peripheral and two central x20 fields of the tumor are captured with a FITC filter and an integrated frame grabber. Only mitotic figures in metaphase-telophase (MI) are included in the mitotic indices to exclude the potential artifact of nuclear membrane distortion. Apoptotic/Pyknotic nuclei are defined as H₂B-GFP labeled nuclei with a cross sectional area <30µm². Nuclear karyorrhexis (NK), easily distinguishable by the vesicular nuclear condensation and brightness of H2BGFP, is included within this apoptotic indices.

Image Analysis of Vascular Parameters: For each spheroid, video recordings are used to calculate length, area and vascular density of the neovasculature being induced by the implanted tumor spheroids. Vascular parameters are analyzed from the video recording using Image-Pro Plus. Photomicrographs obtained with the x10 objective, are "flattened" to reduce the intensity variations in the background pixels. An Area of Interest (AOI) is selected to eliminate distorted areas, and thresholding is used to segment the picture into objects and background. This panel is used to calculate the vascular area (A_{V}). The picture is skeletonized to calculate the vascular length (L_{V}). The average tumor vessel diameter D_{V} is calculated as A_{V}/L_{V}, and the vascular density (Δ_{V}) is calculated as L_{V} per tumor area. Finally, we calculate the growth rate of the total area of tumor vasculature.

### Example 3

### Quantification of cell cycle entry and cell cycle analysis by Flow Cytometry

Prostate cancer cells of interest are plated the day before the experiment. After 24 hours, cells are incubated with normal growth medium and/or medium conditions to be studied (e.g. no-androgen versus androgen) for 24 hours. Cells are then treated with increasing concentrations of one or more test compounds for 24 hours. At the end of the incubation period, cells are suspended using trypsin for 5-10 min, fixed and permeabilized using BD cytofix/cytoperm solution (BD Pharmingen, San Jose, CA) according to instructions provided with the kit. Cells are incubated with antibodies to phospho-histone H3 for 30 min, washed three times in BD perm/wash buffer, and stained with Alexa Fluor-488 anti-mouse antibodies for 20 min followed by three more washes. The cells are re-suspended at a density of approximately 106 cells/0.5 ml in BD perm/wash buffer containing 50 µg/ml DNase-free RNase A, and 50 µg/ml propidium iodine. Fluorescence of single cells is recorded using a flow cytometer (BD Pharmingen, San Jose, CA). FlowJoTM Software is used for data analysis.

For example, TRAMP-C2 cells (transfected with H2B-GFP) were plated at 2.08 x 10⁴ cells/cm² density in complete RPMI medium (10% of heat-inactivated FCS, 10⁻⁸ M DHT, 5 µg/ml insulin, 6 ng/ml EGF, 100 U/ml penicillin, 100 µg/ml streptomycin, 2 mM L-glutamine) and grown at 37° C in a humidified 5% CO₂ atmosphere overnight. The cells were 3X washed with PBS and medium was replaced for RPMI medium without phenol red and without FCS. One set of cells had no DHT, the other set was incubated in the presence of DHT 10⁻⁸ M overnight. 24 h later, the medium was replaced for medium (RPMI without phenol red, no FCS) containing increasing concentrations of plumbagin in the range 0-1 µM with DHT 10⁻⁸ M or without DHT. 24 h later the incidence of MI, AP, and PY was evaluated by fluorescent microscopy. The results of the analysis are provided in Figure 3 and Table 3.

**Table 3:**

| Concentration (µM) | MI (x 10⁻³) | AP (x 10⁻³) | PY (x 10⁻³) |
|---|---|---|---|
| Plumbagin: 0 | 3.25829 | 3.855012 | 7.086273 |
| Plumbagin: 0.4 | 8.889835 | 8.852212 | 20.495 |
| Plumbagin: 0.6 | 14.81127 | 9.370655 | 16.76075 |
| Plumbagin: 0.8 | 12.70411 | 16.10954 | 16.73318 |
| Plumbagin: 1 | 7.284778 | 14.22422 | 8.243806 |
| DHT: 0.01 | 4.911026 | 13.80126 | 0.868056 |

These above-described results indicate that 0.6 µM of plumbagin induces cell cycle entry, and specifically induces mitosis in TRAMP-C2 cells much more effectively than DHT at 10⁻⁸ M. Consequently, this experiment demonstrates that plumbagin is acting as a growth factor to induce mitosis in these cells.

Using the above-described protocol, individual compounds are screened for their ability to act as regulators of cell cycle entry, regulators of entry into specific phases of the cell cycle, or as growth factors, along with their general effectiveness. Additionally, compounds of Formula (I) (i.e., plumbagin) are (A) examined at various concentrations; (B) examined at various concentrations with the administration (pre-treatment, concurrent, or post-treatment) of a selective inhibitor of 17,20-lyase activity of cytochrome P450-17 (CYP17) (i.e, orteronel or VT-464; (C) examined at various concentrations with the administration (pre-treatment, concurrent, or post-treatment) of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl}-3-oxo-4-azaandrost-1-ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18B-glycerrhetinic acid; altraric acid; N-butylbenzene-sulfonamide; 3,3'-diindolylmethane; deslorelin; nafarelin; cetrorelix; or ganirelix; and (D) examined at various concentrations with the administration (pre-treatment, concurrent, or post-treatment) of a selective androgen receptor antagonist, an inhibitor of androgen synthesis, a 5α-reductase inhibitor, or a plant-derived inhibitor.

Optionally, comparative studies are run against one or more of plumbagin in combination with cyproterone acetate, abiraterone, finasteride, flutamide, nilutamide, bicalutamide, ethylstilbestrol (DES), megestrol acetate, fosfestrol, estamustine phosphate, leuprolide, triptorelin, goserelin, histrelin, buserelin, abarelix and/or degarelix.

### Example 4

### Effect of naphthoquinone analog treatment combinations on PTEN-P2/GFP cell proliferation

PTEN-P2/GFP prostate cancer cells are plated at a density of 8000 cells/well in 96-well plates (triplicates) in growing medium containing 10% Fetal Bovine Serum and DHT. The next day, increasing concentrations of a naphthoquinone analog (diluted from 10 mM DMSO stock solutions) are incubated for 24hrs. Cell viability is determined by the formazan-based cytotoxicity assay "CellTiter96Aquaeous nonradioactive proliferation assay" (Promega).

Using the above-described protocol individual compounds of Formula (I) (i.e., plumbagin) are (A) examined at various concentrations; (B) examined at various concentrations with the administration (pre-treatment, concurrent, or post-treatment) of a selective inhibitor of 17,20-lyase activity of cytochrome P450-17 (CYP17) (i.e, orteronel or VT-464; (C) examined at various concentrations with the administration (pre-treatment, concurrent, or post-treatment) of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl}-3-oxo-4-azaandrost-1-ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18B-glycerrhetinic acid; altraric acid; N-butylbenzene-sulfonamide; 3,3'-diindolylmethane; deslorelin; nafarelin; cetrorelix; or ganirelix; and (D) examined at various concentrations with the administration (pre-treatment, concurrent, or post-treatment) of a selective androgen receptor antagonist, an inhibitor of androgen synthesis, a 5α-reductase inhibitor, or a plant-derived inhibitor.

Optionally, comparative studies are run against one or more of plumbagin in combination with cyproterone acetate, abiraterone, finasteride, flutamide, nilutamide, bicalutamide, ethylstilbestrol (DES), megestrol acetate, fosfestrol, estamustine phosphate, leuprolide, triptorelin, goserelin, histrelin, buserelin, abarelix and/or degarelix.

### Example 5

### Dose response for naphthoquinone analog treatment combinations in PTEN-P2/GFP cells

PTEN-P2/GFP mouse cancer cells are placed in androgen withdrawal medium in the presence or absence of DHT (dihydrotestosterone) at a final concentration of 10⁻⁸ M. Test compounds are added at desired concentrations for 24 hours. The absence of DHT simulates surgical or chemical castration. Cells are trypsinized and counted using a Cell Coulter counter Multisizer II, which excludes debris.

Androgen withdrawal medium: DMEM high-glucose phenol-red free, with the following additives: 10% charcoal-treated Fetal Bovine Serum, 25 µg/ml bovine pituitary extract, 5 µg/ml insulin, 6 ng/ml EGF recombinant.

Using the above-described protocol individual compounds of Formula (I) (i.e., plumbagin) are (A) examined at various concentrations; (B) examined at various concentrations with the administration (pre-treatment, concurrent, or post-treatment) of a selective inhibitor of 17,20-lyase activity of cytochrome P450-17 (CYP17) (i.e, orteronel or VT-464; (C) examined at various concentrations with the administration (pre-treatment, concurrent, or post-treatment) of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl}-3-oxo-4-azaandrost-1-ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1 ,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18B-glycerrhetinic acid; altraric acid; N-butylbenzene-sulfonamide; 3,3'-diindolylmethane; deslorelin; nafarelin; cetrorelix; or ganirelix; and (D) examined at various concentrations with the administration (pre-treatment, concurrent, or post-treatment) of a selective androgen receptor antagonist, an inhibitor of androgen synthesis, a 5α-reductase inhibitor, or a plant-derived inhibitor.

Optionally, comparative studies are run against one or more of plumbagin in combination with cyproterone acetate, abiraterone, finasteride, flutamide, nilutamide, bicalutamide, ethylstilbestrol (DES), megestrol acetate, fosfestrol, estamustine phosphate, leuprolide, triptorelin, goserelin, histrelin, buserelin, abarelix and/or degarelix.

### Example 6

### In vivo effect of naphthoquinone analog treatment combinations in the pseudo-orthotopic chamber model for prostate cancer

Platinum chambers are placed in the dorsal skinfold of nude mice by surgery. Two days later, minced prostate tissue from BalbC mice (syngeneic) is grafted into the chambers and allowed to vascularize for 7 to 10 days. Small tumor cell spheroids are implanted into each chamber. PTEN-P2 stably transfected with H2B-GFP fusion protein (PTEN-P2/GFP) cells are used in these experiments. When tumor vascularization is established (about 5-7 days), the animals are either (A) surgically castrated to inhibit androgen production; or (B) allowed to naturally produce androgens. The mice are then treated with drug combinations (including pre-treatment and concurrent treatment to study the effect of timing for various combinations). In some experiments, test compound treatment is 1 mg/kg (DMSO and PEG30%) via intraperitoneal injection, once/day

For example, the above protocol was followed with PTEN-P2 tumor spheroids implanted in dorsal skinfold chambers in nude mice, with either no treatment (control), treatment with bicalutamide (i.e. Casodex) at 10 mg/kg po, once/day, and treatment with bicalutamide (i.e. Casodex) at 10 mg/kg po, once/day in combination with plumbagin 1 mg/kg ip once/day. The results of this experiment are summarized in Figure 2, comparing tumor growth without treatment ("CONTROL"), with Casodex ("CASODEX"), and with plumbagin and Casodex ("COMB"). Figure 2 illustrates that treatment with bicalutamide and a combination treatment with bicalutamide and plumbagin drastically reduces tumor size. Unexpectedly, the combination treatment appears, at first glance, equal to or slightly less effective than treatment with bicalutamide alone. Without wishing to be bound to a particular theory, it is known that bicalutamide slows tumor growth, rather than induces apoptosis of tumor cells. Schweizer et al., Therapeutic Advances in Urology, 4(4), 167-178. As we have discovered, plumbagin induces both mitosis and apoptosis (via several proposed mechanisms). Plumbagin also recognizes the colchicine binding site of tubulin and inhibits in vitro tubulin polymerization. *See* Acharya et al., Biochemistry 2008, 47(3), 7838-45. Given these effects, and in view of plumbagin's other proposed apoptotic mechanisms, compounds that slow tumor growth, in particular compounds that bind to or interact with the androgen receptor, may counteract one or more of plumbagin's apoptotic effects, thereby resulting in a less efficient treatment.

Using the above-described protocol individual compounds of Formula (I) (i.e., plumbagin) are (A) examined at various concentrations; (B) examined at various concentrations with the administration (pre-treatment, concurrent, or post-treatment) of a selective inhibitor of 17,20-lyase activity of cytochrome P450-17 (CYP17) (i.e, orteronel or VT-464; (C) examined at various concentrations with the administration (pre-treatment, concurrent, or post-treatment) of enzalutamide (4-(3-(4-cyano-3-(trifluoromethyl)phenyl)-5,5-dimethyl-4-oxo-2-thioxoimidazolidin-1-yl)-2-fluoro-N-methylbenzamide); ARN-509 (4-(7-(6-cyano-5-(trifluoromethyl)pyridin-3-yl)-8-oxo-6-thioxo-5,7-diazaspiro[3.4]octan-5-yl)-2-fluoro-N-methylbenzamide); vinclozolin ((RS)-3-(3,5-dichlorophenyl)-5-methyl- 5-vinyloxazolidine-2,4-dione); galeterone (17-(1H-benzimidazol-1-yl)androsta-5,16-dien-3β-ol); ketoconazole (1-[4-(4-{[(2R,4S)-2-(2,4-Dichlorophenyl)-2-(1H-imidazol-1-ylmethyl)-1,3-dioxolan-4-yl]methoxy}phenyl)piperazin-1-yl]ethan-1-one); L-39 (17-(5'-Isoxazolyl)androsta-4,16-dien-3-one); aminoglutethimide; prochloraz (N-propyl-N-[2-(2,4,6-trichlorophenoxy)ethyl]imidazole-1-carboxamide); dutasteride (5a, 17β)-N-{2,5 bis(trifluoromethyl) phenyl} -3-oxo-4-azaandrost-1-ene-17-carboxamide); izonteride ((4aR,10bR)-8-[(4-ethyl-1,3-benzothiazol-2-yl)sulfanyl]-4,10b-dimethyl-1,4,4a,5,6,10b-hexahydrobenzo[f]quinolin-3(2H)-one); turosteride ((4aR,4bS,6aS,7S,9aS,9bS,11aR)-1,4a,6a-trimethyl-2-oxo-N-(propan-2-yl)-N-(propan-2-ylcarbamoyl)hexadecahydro-1H-indeno[5,4-f]quinoline-7-carboxamide); epristeride (17-(tert-butylcarbamoyl)androsta-3,5-diene-3-carboxylic acid); genisterin; gossypol; equol; 18β-glycerrhetinic acid; altraric acid; N-butylbenzene-sulfonamide; 3,3'-diindolylmethane; deslorelin; nafarelin; cetrorelix; or ganirelix; and (D) examined at various concentrations with the administration (pre-treatment, concurrent, or post-treatment) of a selective androgen receptor antagonist, an inhibitor of androgen synthesis, a 5α-reductase inhibitor, or a plant-derived inhibitor.

Optionally, comparative studies are run against one or more of plumbagin in combination with cyproterone acetate, abiraterone, finasteride, flutamide, nilutamide, ethylstilbestrol (DES), megestrol acetate, fosfestrol, estamustine phosphate, leuprolide, triptorelin, goserelin, histrelin, buserelin, abarelix and/or degarelix.

### Example 7

### Animal Model and Surgical Techniques

Experiments were approved by the appropriate Institutional Animal Care and Use Committee (IACUC) and were carried out according to National Institutes of Health (NIH) recommended procedures and precautions. Surgeries were performed in a sterile laminar flow hood. Platinum chambers and surgical instruments were autoclaved before use. Saline used to keep tissue moist during surgical preparation was mixed with gentamicin (50 ml/ml). Platinum chambers were fitted in the dorsal skinfold of male nude mice by surgery as described extensively in Frost et al., "The roles of epithelial-mesenchymal interactions and the innate immune response on the tumorigenicity of human prostate carcinoma cell lines grown in immuno-compromised mice," In vivo (Athens, Greece) 2003, 17(5):377-388; Frost et al., "Novel syngeneic pseudo-orthotopic prostate cancer model: vascular, mitotic and apoptotic responses to castration," Microvasc. Res. 2005, 69(1/2): 1-9; and Oh et al., "Live dynamic imaging of caveolae pumping targeted antibody rapidly and specifically across endothelium in the lung," Nat. Biotechnol. 2007, 25(3):327-337. Two days later, a BalbC male donor mouse was euthanized and the anterior prostate tissue was excised, placed in a Petri dish with gentamicin (50 ml/ml), and minced with fine scissors into small pieces (<1 mm²) that were implanted into the dorsal chambers of host mice. The grafted tissue was allowed to vascularize for 7-10 days. Mouse cancer cells PTEN-P2/H2B-GFP grown as pre-confluent monolayers were trypsinized and suspended in a final volume of 250,000 tumor cells/ml. Viability was determined using Trypan blue and a small number of cells (typically 50,000) were centrifuged in micro titer plates forming spheroids, each of which was placed on top of the grafted prostate tissue (one spheroid/chamber).

Surgical castration was performed on mice anesthetized with 7.3 mg ketamine hydrochloride and 2.3 mg xylazine/100 g body weight, i.p. A lateral incision across the scrotum was made and the testes were individually ligated and excised. The wound was cauterized. The incision was then sutured and sealed with Nexaband1 acrylic. Surgical castration induces androgen deprivation, and mimics clinical hormone therapy.

Plumbagin was formulated as an inclusion complex with heptakis(2,6-di-O-methyl)-beta-cyclodextrin. In brief, a super-saturated solution of plumbagin in 0.1M of heptakis(2,6-di-O-methyl)-beta-cyclodextrin was stirred for 72 hours at 25+/-0.5 °C. Then the mixture was filtered through a 0.2 µm nylon membrane filter. The concentration of solubilized plumbagin has been determined by UV-VIS spectroscopy (Amax(263 nm and 417 nm)). Phase-solubility diagram analysis showed K1:1 = 485 M⁻¹.

TAK-700 (Orteronel) was formulated as an inclusion complex with sulfobutyl ether-beta-cyclodextrin. In brief, 100 mg of TAK-700 was suspended and stirred in 19.5 ml of 50 mM solution of sulfobutyl ether-beta-cyclodextrin for 24 h at 25 °C. Then the mixture was filtered through a 0.2 µm nylon membrane filter and the concentration of solubilized TAK-700 was determined by UV-VIS spectroscopy.

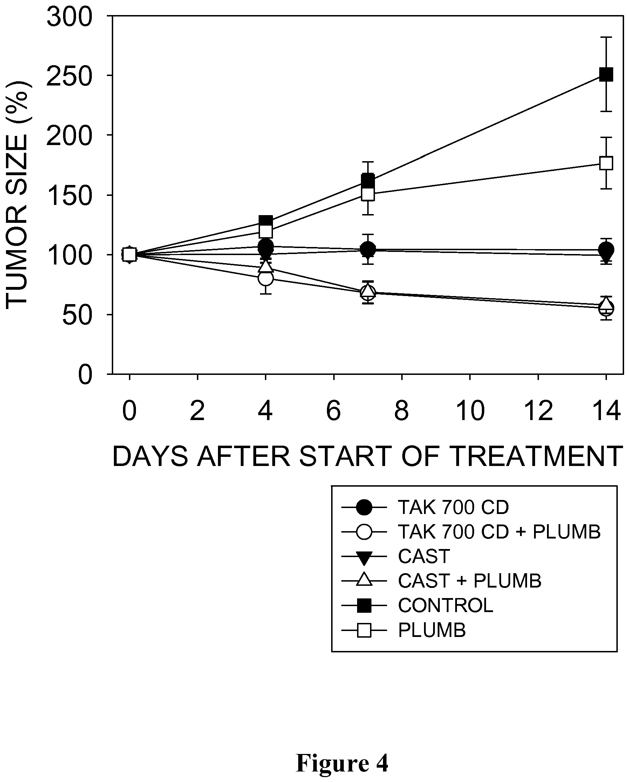

The above protocol was followed with PTEN-P2 tumor spheroids implanted in dorsal skinfold chambers in nude mice, with either no treatment ("CONTROL"), treatment with the plumbagin formulation at 1 mg/kg po, once/day ("PLUMB"), treatment with the TAK-700 formulation at 6 mg/kg po, twice/day ("TAK 700 CD"), treatment with a combination of the TAK-700 formulation at 6 mg/kg po, twice/day and the plumbagin formulation at 1 mg/kg po, once/day ("TAK 700 CD + PLUMB"), treatment with castration ("CAST"), and treatment with castration and the plumbagin formulation at 1 mg/kg po, once/day ("CAST + PLUMB"). The results of this experiment are summarized in Figure 4, which demonstrates that the combination of TAK-700 and plumbagin drastically and unexpectedly reduces tumor size. A comparison of the data shown in Figures 2 and 4 further reveals that the combination of TAK-700 with plumbagin significantly reduces tumor size more effectively than the combination of Casodex and plumbagin.

## Claims

1. A product combination for use in treating or inhibiting androgen-dependent prostate cancer and/or inhibiting or delaying the onset of castration-resistant prostate cancer (CRPC) in a subject, wherein the product combination comprises:
a first compound that is dutasteride (5a, 17β)-N-{2,5 bis(trifluoromethyl) phenyl}-3-oxo-4-azaandrost-1-ene-17-carboxamide); and
a second compound of Formula (I) or a pharmaceutically acceptable salt of Formula (I): 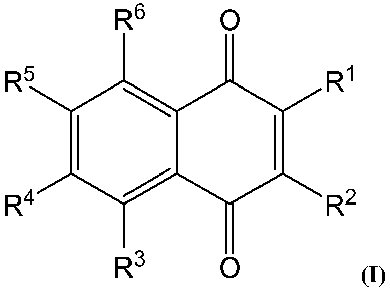 wherein:
R¹ is methyl;
R² is hydrogen;
R³ is -OH;
R⁴ is hydrogen;
R⁵ is hydrogen; and
R⁶ is hydrogen.

2. The product combination of Claim 1, wherein the compound of Formula (I) is administered to the patient before, during, or after administration of dutasteride (5a, 17β)-N-{2,5 bis(trifluoromethyl)phenyl}-3-oxo-4-azaandrost-1-ene-17-carboxamide).

3. The product combination of Claim 1, wherein the patient's serum testosterone level is reduced to about 5-20%, 10-30%, 20-40%, 30-50%, 40-60%, or 50-70% of a healthy male patient; or
wherein the patient's serum testosterone level is reduced to at least about ≤ 20 ng/dL.

4. The product combination of Claim 1, wherein said product combination results in a decrease in prostate cancer tumor size; or
wherein said product combination inhibits the growth of prostate cancer; or
wherein said product combination inhibits or delays the onset of castration-resistant prostate cancer.

5. The product combination of Claim 1, wherein the compound of formula (I) is formulated for oral administration; and/or
wherein dutasteride (5a, 17β)-N-{2,5 bis(trifluoromethyl)phenyl}-3-oxo-4-azaandrost-1-ene-17-carboxamide) is formulated for oral administration.

6. The product combination of claim 1, wherein said product combination induces cell death, cell cycle entry, mitosis, or apoptosis of androgen-dependent cancer cells in a patient.

7. The product combination of Claim 6, wherein the compound of Formula (I) is provided to said patient before, during, or after administration of dutasteride (5a, 17β)-N- {2,5 bis(trifluoromethyl)phenyl}-3-oxo-4-azaandrost-1-ene-17-carboxamide).

8. The product combination of Claim 6, wherein the patient's serum testosterone level is reduced to about 5-20%, 10-30%, 20-40%, 30-50%, 40-60%, or 50-70% of a healthy male patient; or
wherein the subject's serum testosterone level is reduced to at least about ≤ 20 ng/dL.

9. The product combination of Claim 6, wherein said product combination inhibits the growth of prostate cancer; or
wherein said product combination inhibits or delays the onset of castration-resistant prostate cancer.

10. The product combination of Claim 6, wherein the inducement of cell cycle entry, mitosis, or cell death is measured.

11. The product combination of Claim 10, wherein the measurement is made by pyknosis.

## Patentansprüche

1. Produktkombination zur Verwendung beim Behandeln oder Hemmen von androgenabhängigem Prostatakrebs und/oder Hemmen oder Verzögern des Beginns von kastrationsresistentem Prostatakrebs (CRPC) bei einem Subjekt, wobei die Produktkombination Folgendes umfasst:
eine erste Verbindung, bei der es sich um Dutasterid (5a,17ß)-N-{2,5-bis(trifluormethyl)phenyl}-3-oxo-4-azaandrost-1-en-17-carboxamid) handelt; und
eine zweite Verbindung der Formel (I) oder ein pharmazeutisch akzeptables Salz der Formel (I): 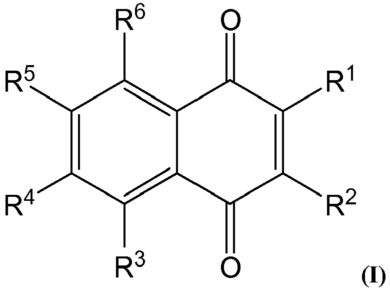 wobei:
R¹ Methyl ist;
R² Wasserstoff ist;
R³ -OH ist;
R⁴ Wasserstoff ist;
R⁵ Wasserstoff ist; und
R⁶ Wasserstoff ist.

2. Produktkombination nach Anspruch 1, wobei die Verbindung der Formel (I) dem Patienten vor, während oder nach der Verabreichung von Dutasterid (5a,17ß)-N-{2,5-bis(trifluormethyl)phenyl}-3-oxo-4-azaandrost-1-en-17-carboxamid) verabreicht wird.

3. Produktkombination nach Anspruch 1, wobei der Serumtestosteronspiegel des Patienten auf etwa 5-20 %, 10-30 %, 20-40 %, 30-50 %, 40-60 % oder 50-70 % eines gesunden männlichen Patienten reduziert ist; oder
wobei der Serumtestosteronspiegel des Patienten auf mindestens etwa ≤ 20 ng/dl reduziert ist.

4. Produktkombination nach Anspruch 1, wobei die Produktkombination zu einer Verringerung der Tumorgröße des Prostatakrebses führt; oder
wobei die Produktkombination das Wachstum des Prostatakrebses hemmt; oder
wobei die Produktkombination den Beginn von kastrationsresistentem Prostatakrebs hemmt oder verzögert.

5. Produktkombination nach Anspruch 1, wobei die Verbindung der Formel (I) zur oralen Verabreichung formuliert ist; und/oder
wobei Dutasterid (5a,17ß)-N-{2,5-bis(trifluormethyl)phenyl}-3-oxo-4-azaandrost-1-en-17-carboxamid) zur oralen Verabreichung formuliert ist.

6. Produktkombination nach Anspruch 1, wobei die Produktkombination Zelltod, Zellzykluseintritt, Mitose oder Apoptose von androgenabhängigen Krebszellen bei einem Patienten induziert.

7. Produktkombination nach Anspruch 6, wobei die Verbindung der Formel (I) dem Patienten vor, während oder nach der Verabreichung von Dutasterid (5a,17ß)-N-{2,5-bis(trifluormethyl)phenyl}-3-oxo-4-azaandrost-1-en-17-carboxamid) bereitgestellt wird.

8. Produktkombination nach Anspruch 6, wobei der Serumtestosteronspiegel des Patienten auf etwa 5-20 %, 10-30 %, 20-40 %, 30-50 %, 40-60 % oder 50-70 % eines gesunden männlichen Patienten reduziert ist; oder
wobei der Serumtestosteronspiegel des Subjekts auf mindestens etwa ≤ 20 ng/dl reduziert ist.

9. Produktkombination nach Anspruch 6, wobei die Produktkombination das Wachstum des Prostatakrebses hemmt; oder
wobei die Produktkombination den Beginn von kastrationsresistentem Prostatakrebs hemmt oder verzögert.

10. Produktkombination nach Anspruch 6, wobei die Induzierung von Zellzykluseintritt, Mitose oder Zelltod gemessen wird.

11. Produktkombination nach Anspruch 10, wobei die Messung durch Pyknose erfolgt.

## Revendications

1. Combinaison de produits pour utilisation dans le traitement ou l'inhibition du cancer de la prostate dépendant des androgènes et/ou l'inhibition ou le retard de l'apparition du cancer de la prostate résistant à la castration (CPRC) chez un sujet, dans laquelle la combinaison de produits comprend :
un premier composé qui est le dutastéride (5a, 17β)-N-{2,5 bis(trifluorométhyl)phényl}-3-oxo-4-azaandrost-1-ène-17-carboxamide) ; et
un deuxième composé de Formule (I) ou un sel pharmaceutiquement acceptable de Formule (I) : 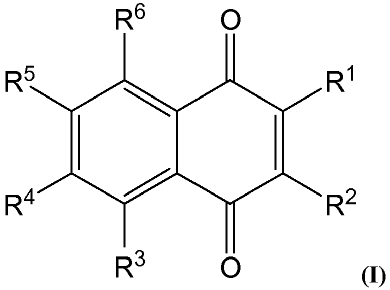 dans laquelle :
R¹ est un méthyle ;
R² est un hydrogène ;
R³ est -OH ;
R⁴ est un hydrogène ;
R⁵ est un hydrogène ; et
R⁶ est un hydrogène.

2. Combinaison de produits selon la revendication 1, dans laquelle le composé de Formule (I) est administré au patient avant, pendant, ou après l'administration de dutastéride (5a, 17β)-N-{2,5 bis(trifluorométhyl)phényl}-3-oxo-4-azaandrost-1-ène-17-carboxamide).

3. Combinaison de produits selon la revendication 1, dans laquelle le taux sérique de testostérone du patient est réduit à environ 5 à 20 %, 10 à 30 %, 20 à 40 %, 30 à 50 %, 40 à 60 %, ou 50 à 70 % d'un patient mâle sain ; ou
dans laquelle le taux sérique de testostérone du patient est réduit à au moins environ ≤ 20 ng/dL.

4. Combinaison de produits selon la revendication 1, dans laquelle ladite combinaison de produits entraîne une diminution de la taille d'une tumeur de cancer de la prostate ; ou
dans laquelle ladite combinaison de produits inhibe la croissance du cancer de la prostate ; ou
dans laquelle ladite combinaison de produits inhibe ou retarde l'apparition du cancer de la prostate résistant à la castration.

5. Combinaison de produits selon la revendication 1, dans laquelle le composé de Formule (I) est formulé pour une administration par voie orale ; et/ou
dans laquelle le dutastéride (5a, 17β)-N-{2,5 bis(trifluorométhyl) phényl}-3-oxo-4-azaandrost-1-ène-17-carboxamide) est formulé pour une administration par voie orale.

6. Combinaison de produits selon la revendication 1, dans laquelle ladite combinaison de produits déclenche la mort cellulaire, l'entrée dans le cycle cellulaire, la mitose, ou l'apoptose de cellules de cancer dépendant des androgènes chez un patient.

7. Combinaison de produits selon la revendication 6, dans laquelle le composé de Formule (I) est fourni audit patient avant, pendant, ou après l'administration de dutastéride (5a, 17β)-N-{2,5 bis(trifluorométhyl)phényl}-3-oxo-4-azaandrost-1-ène-17-carboxamide).

8. Combinaison de produits selon la revendication 6, dans laquelle le taux sérique de testostérone du patient est réduit à environ 5 à 20 %, 10 à 30 %, 20 à 40 %, 30 à 50 %, 40 à 60 %, ou 50 à 70 % d'un patient mâle sain ; ou
dans laquelle le taux sérique de testostérone du sujet est réduit à au moins environ ≤ 20 ng/dL.

9. Combinaison de produits selon la revendication 6, dans laquelle ladite combinaison de produits inhibe la croissance du cancer de la prostate ; ou
dans laquelle ladite combinaison de produits inhibe ou retarde l'apparition du cancer de la prostate résistant à la castration.

10. Combinaison de produits selon la revendication 6, dans laquelle le déclenchement de l'entrée dans le cycle cellulaire, la mitose, ou la mort cellulaire est mesuré.

11. Combinaison de produits selon la revendication 10, dans laquelle la mesure est faite par pycnose.
